# EUROPEAN PATENT SPECIFICATION

(11) **EP 1 024 822 B1**
(45) Date of publication and mention of the grant of the patent: **10.04.2002**
(21) Application number: 98950218.2
(22) Date of filing: 28.10.1998
(51) Int. Cl.: A61K 38/17, A61P 37/06, C07K 14/705, C12N 15/12

(54) **CD8 AS AN INHIBITOR OF THE CELLULAR IMMUNE SYSTEM**
CD8 ALS INHIBITOR DES ZELLULÄREN IMMUNSYSTEMS
CD8 INHIBITRICES DU SYSTEME IMMUNITAIRE CELLULAIRE

(30) Priority: 28.10.1997 GB 9722779
(43) Date of publication of application: 09.08.2000
(73) Proprietor: Avidex Ltd, Abingdon, Oxon OX14 4RX (GB)
(72) Inventor: JAKOBSEN, Bent, Karsten, John Radcliffe Hospital, Oxford, Oxfordshire OX3 9DS (GB); GAO, George, Fu, Lab. of Molecular Medicine, 320 Longwood Avenue, Boston, MA 02115 (US); GERTH, Ulrich, Conrad, Hypo Vereinsbank, D-81925 München (DE); SEWELL, Andrew, Kelvin, John Radcliffe Hospital, Oxford, Oxfordshire OX3 9DU (GB)
(74) Representative: Lee, Nicholas John
(86) International application number: GB9803235
(87) International publication number: WO9921576

(56) References cited:
- WO-A-96/22106
- PALIWAL V ET AL: "Recombinant soluble alphabeta T cell receptors protect T cells from immune suppression: requirement for aggregated multimeric, disulfide-linked alphabeta heterodimers." JOURNAL OF IMMUNOLOGY, (1997 AUG 15) 159 (4) 1718-27, XP002093583
- GAO G F ET AL: "Crystal structure of the complex between human CD8alpha(alpha) and HLA-A2." NATURE, (1997 JUN 5) 387 (6633) 630-4, XP002093584 cited in the application
- BOURSIER J P ET AL: "Evidence for an extended structure of the T-cell co-receptor CD8 alpha as deduced from the hydrodynamic properties of soluble forms of the extracellular region." JOURNAL OF BIOLOGICAL CHEMISTRY, (1993 JAN 25) 268 (3) 2013-20, XP002093585 cited in the application
- GAO, GEORGE F. ET AL: "Assembly and crystallization of the complex between the human T cell coreceptor CD8alpha homodimer and HLA-A2." PROTEIN SCIENCE, (MAY, 1998) VOL. 7, NO. 5, PP. 1245-1249, XP002093586
- JELONEK M T ET AL: "Direct binding of the MHC class I molecule H-2Ld to CD8: interaction with the amino terminus of a mature cell surface protein." JOURNAL OF IMMUNOLOGY, (1998 MAR 15) 160 (6) 2809-14, XP002093587

## Description

This invention relates to uses as defined in the claims for inhibiting immune responses, in particular cellular immune responses involving CD8 positive lymphocytes, using soluble (or non-cellular) forms of CDB. The invention also relates to compositions comprising soluble forms of CD8 and to multimers of the soluble CD8 as defined in the claims.

### INTRODUCTION

Major histocompatibility complex Class I and II proteins (MHC, or HLA in man) bind peptide antigens and present them on the cell surface for recognition by T lymphocytes expressing a unique T cell receptor (TCR) matching the specific MHC-peptide combination. The transmembrane glycoproteins CD8 and CD4 are characteristic of distinct populations of T lymphocytes whose antigen responses are restricted by class I and class II MHC molecules, respectively. CD8 and CD4 play major roles both in the differentiation and selection of T cells during thymic development and in the activation of mature T lymphocytes in response to antigen presenting cells. CD8 and CD4 are therefore considered to be the main accessory molecules for T cell receptors. Although CD8 and CD4 are immunoglobulin superfamily proteins and determine antigen restriction by binding to MHC molecules, but not to the antigenic peptide, the structural basis for their similar functions appears to be very different. Their sequence similarity is low and whereas CD4 is expressed on the cell surface as a monomer CD8 is expressed as an αα homodimer or an αβ heterodimer.

CD8 expression is characteristic of cytotoxic T lymphocytes (CTL) and important for their progression through the process of 'positive selection' during differentiation in the thymus. In mature cytotoxic lymphocytes the immunological role of CD8 is complex and, as yet, not fully clarified. From the current understanding three putative roles in CTL function can be envisaged. Firstly, CD8 plays a role in T cell signalling. Secondly, a significant function of CD8 may be to assist in cell-cell adhesion, tethering the T cell to the antigen presenting cell by increasing the avidity of the interaction. In most, but not all, mature cytotoxic lymphocytes target cell lysis can be blocked by anti-CD8 antibodies. Thirdly, CD8 may serve as a coreceptor for TCR involving a mechanism for collaborative binding of the two receptors to the MHC/peptide ligand. Comparison of the structures of the TCR-MHC and CD8-MHC complexes provide clues for how this cooperativity could work. Although CD8 imposes no detectable conformational changes on the peptide loaded cleft of the MHC molecule, where the TCR binds, it does introduce a shift in the MHC α3 domain which is similar to a shift introduced by TCR binding. However, analysis involving a human TCR does not indicate that binding is energetically favoured in the presence of CD8.

The T cell coreceptor CD8 is essential for the positive selection of cytotoxic T-lymphocytes (CTL) during differentiation in the thymus and for the ability of most mature CTLs to kill target cells. CDB, expressed on the T cell surface as αα or *αβ* dimers, contacts the MHC class I molecules or more specifically a nonpolymorphic region of the MHC class I α3 domain, on antigen presenting cells, thereby increasing the avidity of the T cell for its target. CD8 is also involved in the phosphorylation events leading to T cell activation through the association of the α chain cytoplasmic tail with p56^{*lck*}.

The analysis of the molecular basis underlying cellular immune responses has suffered from the lack of availability of soluble protein reagents with which to study structural and kinetic aspects of the interactions involved. Recently however a number of groups using expression systems ranging from E.coli to mammalian cells have reported strategies for obtaining soluble TCR. Similarly, a number of groups have reported strategies for obtaining soluble CD8.

Leahy *et al* (1992) describe the crystallisation of a human soluble CD8α molecule produced as a secreted disulphide-linked homodimer in CHO cells. The CD8α contains residues 1 to 146 of the mature protein. In an attempted version employing residues 1 to 114 only, no detectable secreted protein was produced.

Boursier *et al* (1993) describe the production of a soluble CD8 protein which is expressed as a soluble fusion protein in *E*.*coli*. One form consisted of residues 1 to 114 of the mature protein, that is the immunoglobulin domain of the protein. Functionality of the soluble protein is not demonstrated.

Garcia *et al* (1996) describe a soluble CD8 expressed in insect cells. The CD8 molecule contained most of the extracellular part of the CD8 molecule, at least residues 1 to 146 of the mature CD8 protein. Preliminary binding studies are reported.

WO 96/22106 and Choski *et al* (1998) disclose peptide fragments of murine CD8 purported to inhibit T cells. The peptides are used at a very high concentration of 100 µg/ml. No evidence is given to show whether or not the peptides are actually binding to the MHC molecules.

### THE INVENTION

The invention is based on the surprising discovery that soluble forms of CD8 strikingly inhibit CTL activity, both *in vitro* and *in vivo*. Given that CD8 is merely a co-receptor, and given the known low affinity of CD8 for MHC (Garcia *et al* 1996), this was entirely unexpected. It has been found that binding of soluble CD8 to less than 1% of MHC molecules on target cells is sufficient for inhibition. The effect is explained at least in part by a complete inhibition of the early signalling events in the T cell. This effect bears similarity to the phenomenon of "peptide antagonism" in which a small ratio of mutated peptide ligand can switch off T cells. It is believed that in a similar fashion, the prevention of just a few interactions between the receptors on the T cells and the MHC on the target cells may have a dramatic effect by disrupting or destabilising the multimeric signalling complex in the T cell. In addition, the inhibition effect is observed to occur strongly at concentrations of soluble CD8 far lower than the peptide concentrations of Choski *et al* (cited above)

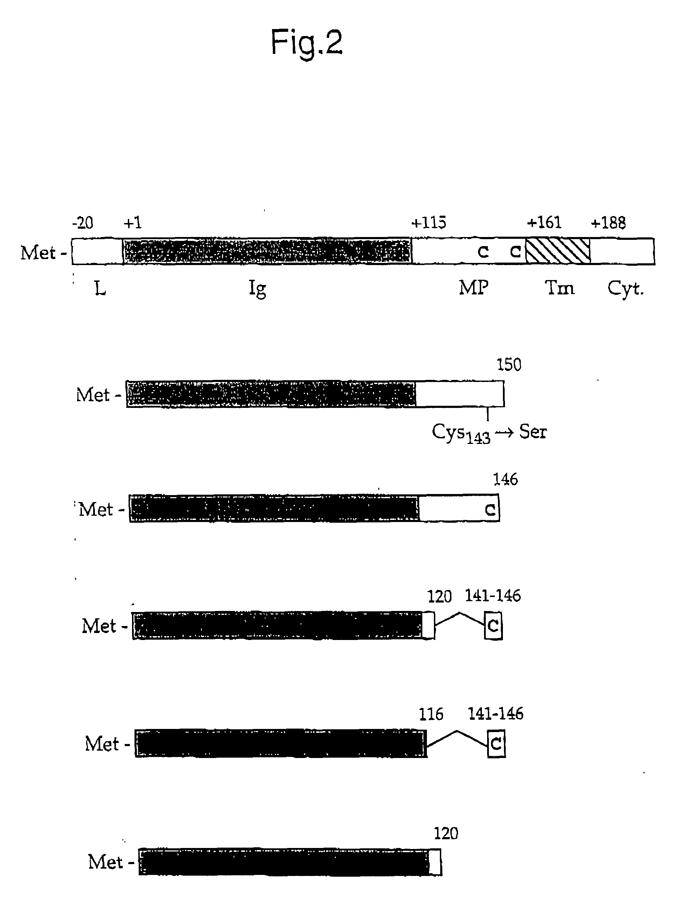

A strategy for overexpression in *E.coli* of the extracellular immunoglobulin domain of human CD8α was devised. Codon usage alterations in the 5' region of the gene were designed so as to prevent the formation of secondary structures in the mRNA. A fragment of CD8α, comprising residues 1-120 of the mature protein thus excluding the signal peptide, the cytoplasmic and transmembrane domains and part of the membrane-proximal stalk region, was recovered from bacterial inclusion bodies and refolded to produce a single species of homodimeric, soluble receptor. Figure 2 illustrates the domain structure of CD8 as described above, and showing other truncations of the CD8α protein which were expressed but did not fold correctly. HLA-A2 heavy chain and β2-microglobulin were similarly expressed and refolded with a synthetic peptide antigen corresponding to the *pol* epitope from HIV-1. CD8α/HLA-A2 complexes were formed in solution and by co-crystallization with a stoichiometry of one CD8αα dimer to one HLA-A2-peptide unit.

Data is herein given demonstrating that a fragment of the CD8αα receptor is capable of inhibiting T cell cytotoxicity, and that mutations of the CD8αα fragment can modulate that capability. The inhibitory activity of the soluble CD8 is striking both *in vitro* and *in vivo*. Also described herein is the above strategy for high-level expression of the extracellular immunoglobulin domain of human CD8α in *E.coli* and for the folding of the CD8αα dimer as a MHC-binding receptor. Also described is the assembly and crystallisation of the complex between CD8αα and HLA-A2 associated with peptide antigen.

In one aspect, the invention provides the use of a soluble CD8αα or αβ molecule which comprises a correctly-folded immunoglobulin domain in the manufacture of a medicament for immunosuppressive therapy.

In another aspect, the invention provides the use of a multimer of two or more soluble CD8αα or αβ molecules in the manufacture of a medicament for immunosuppressive therapy.

The CD8 molecule may be any suitable soluble CD8 molecule which has a functional immunoglobulin domain, preferably human. Generally this will comprise all or a substantial part of the native CD8 immunoglobulin domain, optionally substituted at one or more amino acid residues, particularly substitutions which increase the binding affinity of the soluble CD8 for MHC. The soluble CD8 may further comprise a fragment of the membrane-proximal stalk region of CD8, and may comprise residues 1-120 of mature CD8.

The term "soluble form" is used herein in relation to the CD8 molecule in the manner in which it is conventionally used in the an in relation to cell surface receptors. A soluble form of a cell surface receptor is usually derived from the native form by deletion of the transmembrane domain. The protein may be truncated by removing both the cytoplasmic and the transmembrane domains, or there may be deletion of just the transmembrane domain with part or all of the cytoplasmic domain being retained. The important thing is that the desired extracellular function of the receptor is retained, which is in this case the MHC-binding capability of the CD8 immunoglobulin domain. The protein may be modified to achieve the desired form by proteolytic cleavage, or by expressing a genetically engineered truncated or partially deleted form.

The invention finds particular uses in the treatment of patients requiring immunosuppressive therapy. Such patients include transplant patients, either awaiting transplant, undergoing transplantation or after transplantation has taken place. Autoimmune diseases and allergies may also usefully be treated by immunosuppressive therapy. One specific example is exacerbated asthma in which T cells come into play as a result of viral infection. Severe damage to the lungs follows and the result is chronic asthma which can lead to death. The current treatment is with corticosteroids which strongly suppress the immune system. A preferable treatment would be one which suppresses the immune system more selectively, such as specific blocking of CTL function by soluble CD8 as described herein.

Thus, the invention provides in another aspect a composition comprising a soluble form of a CD8αα or αβ molecule which comprises a correctly-folded immunoglobulin domain, together with a pharmaceutically acceptable diluent, excipient or carrier, for immunosuppressive therapy.

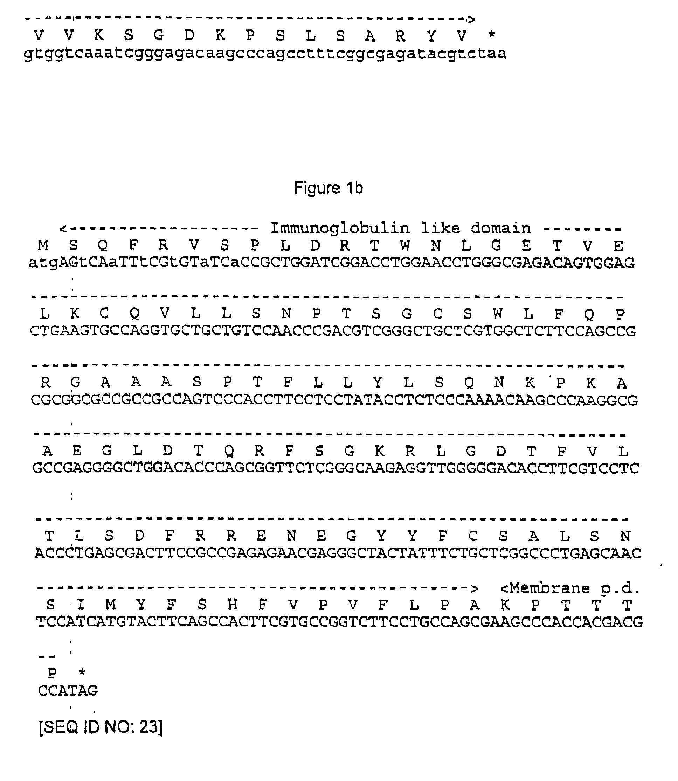

The invention provides in still another aspect, a particular soluble CD8 protein having the sequence shown in figure 1b, said protein folded as a dimer and having the property of inhibiting the action of cytotoxic T cell lymphocytes to kill target cells.

In a further aspect the invention provides a soluble CD8 protein which differs from the specific protein defined above, in one or more of the following respects: i) methionine absent at the N-terminus; ii) 1-15 amino acid residues absent from the N-terminus; iii) part or all of the sequence 'leu - leu - leu - his - ala - ala - arg - pro -' (the signal sequence) added at the N-terminus; iv) 1-15 amino acid residues absent from the C-terminus, but with at least a part of the membrane-proximal stalk region i.e. the region defined by amino acids 116-120, retained; v) part or all of the sequence '- ala - pro - arg - pro - pro -thr - pro - ala' added at the C-terminus; vi) conservative variants of one or many amino acid residues which do not materially affect the CD8 functionality of the protein; vii) mutations which do alter the CD8 functionality in such a way that they increase the property of inhibiting the action of cytotoxic T cell lymphocytes to kill target cells; viii) the addition of a protein or peptide, at the N or C terminus for the purposes of purification; ix) the provision of a label for detection; said protein folded as a dimer and having the property of inhibiting the action of cytotoxic T cell lymphocytes to kill target cells.

More particularly, this aspect of the invention provides a soluble CD8 molecule containing a substantial part of the extracellular region of CD8, including the immunoglobulin domain and a fragment of the membrane proximal stalk region, which CD8 molecule is not disulphide-linked between the two chains of the molecule.

In another aspect, the invention provides a complex of a soluble CD8 protein as herein defined with HLA - A2. A further form of this complex is provided in crystalline form with a stoichiometry of one protein dimer to one HLA - A2 peptide unit.

The invention provides, in a still further aspect, a method of producing a recombinant protein as herein described, which method comprises the steps of: i) providing a CD8 derived gene which codes for a CD8 protein as described herein in a bacterium; ii) effecting expression of said CD8 derived gene in said bacterium and recovering the expressed protein from a bacterial culture; iii) treating the expressed protein to facilitate its purification and carrying out said purification. Preferably the CD8 derived gene provided at step i) is modified via silent mutations designed to increase expression via the prevention of the formation of a 5' hairpin secondary structure in the expressed mRNA. Preferably at step iii) the treatment of the expressed protein involves solubilising the protein and treating the protein so as to cause it to fold into a form resembling its native state, which is then purified. Preferably the CD8 derived gene product corresponds to the immunoglobulin-like and membrane-proximal stalk regions of a CD8 protein.

The soluble CD8 molecules described herein may be in the form of a multimer, that is two or more soluble CD8 molecules linked together to produce a bi-functional or multifunctional species. The CD8 molecules may be associated with one another via a linker molecule. Alternatively or additionally the soluble CD8 may be attached to larger entities such as membrane structures or particles. Thus, the invention provides in a further aspect, a multimer of soluble CD8, comprising two or more CD8αα or αβ molecules attached to one another via a linker molecule.

Suitable linker molecules include multivalent attachment molecules such as avidin, streptavidin and extravidin each of which has four binding sites for biotin. Thus, biotinylated CD8 molecules can be formed into multimer complexes of CD8 having a plurality of CD8 binding sites. The number of CD8 molecules in the resulting complex will depend upon the quantity of CD8 in relation to the quantity of linker molecule used to make the complexes, and also on the presence or absence of any other biotinylated molecules. Preferred complexes are trimeric or tetrameric CD8 complexes.

Suitable structures for attachment of soluble CD8, optionally already in multimeric form, include membrane structures such as liposomes and solid structures which are preferably particles such as beads. Other structures which may be externally coated with CD8 molecules are also suitable.

Multimeric forms of CD8 particularly useful in the method according to the invention include multimers formed by linking biotinylated soluble CD8 via e.g. streptavidin as the linker molecule. One or both chains of the CD8 molecule are biotinylated, conveniently by means of a biotinylation sequence expressed as a tag on the α or β chain. Also of particular use are liposome coupled soluble CD8 molecules, in which the CD8 is advantageously itself in the form of a multimer which multimer is then attached to the liposomal membrane by suitable means.

The soluble CD8 will be administered in a manner appropriate for the condition to be treated or prevented. For example, for prevention of graft rejection one or more injections into the local area concerned may be most suitable. On the other hand for an autoimmune disease where the effects are throughout the body it may be more appropriate to inject the soluble CD8 directly into the bloodstream.

Suitable compositions and dosage of soluble CD8 as an immunosuppressive agent can be devised by one of ordinary skill in the art. Two or more doses of a smaller amount of soluble CD8 may be preferable to a single high level dose. Formulations may be for example liquid formulations, or powder formulations such as those designed for delivery by a high velocity needleless delivery device.

The compositions according to the invention may further comprise, or be administered in a treatment regime with, other agents in particular other immunosuppressive agents.

The most commonly used immunosuppressive drugs currently include corticosteroids and more potent inhibitors like, for instance, methotrexate, sulphasalazine, hydroxychloroquine, 6-MP/azathioprine and cyclosporine (Baert and Rutgeerts, 1997; Singer and McCune, 1998). All of these treatments have severe side-effects related to toxicity, however, and the need for drugs that would allow their elimination from, or reduction in use is urgent (McKendry, 1997; Ortiz *et al*., 1998; Sibilia *et al*., 1998; Singer and McCune, 1998). Many immunosuppressive drugs have been found to have greater efficacy when used in combination, even when the total dose is lowered (Verhoeven *et al*., 1998).

Other immunosuppressive drugs include the gentler, but less powerful non-steroid treatments such as Aspirin and Ibuprofen, and a new class of reagents which are based on more specific immune modulator functions. This latter class includes interleukins, cytokines, recombinant adhesion molecules and monoclonal antibodies (for reviews see Baert and Rutgeerts, 1997; Chatenoud, 1998).

sCD8αα is specific for class I MHC molecules and is therefore expected to inhibit only the response of cytotoxic or memory T cells to target cells presenting class I complexes. Many cellular immune responses are of a composite nature, involving class I and class II-restricted T cells. In some situations it may be desirable to use sCD8αα on its own to suppress unwanted CTL responses. In many situations sCD8αα may be useful in combination with other immunosuppressive drugs or reagents which suppress other elements of the immune response.

It is envisaged that including sCD8αα in an immunosuppressive treatment protocol will increase the efficiency of immunosuppression, and particularly, may enable the administered amounts of other drugs, which have toxic or other adverse effects to be decreased.

### 1. Structure of the CD8αα receptor.

The human CD8 gene expresses a protein of 235 amino acids. As illustrated in figure 2, the organisation of the protein can be divided into the following domains (starting at the amino terminal and ending at the carboxy terminal of the polypeptide):
a. signal peptide (amino acids -21 to -1) - this is cleaved off in human cells during the transport of the receptor to the cell surface and thus does not constitute part of the mature, active receptor;
b. immunoglobulin (lg) -like domain (approximately amino acids 1-115) - this domain assumes a structure, referred to as the immunoglobulin fold, which is similar to those of many other molecules involved in regulating the immune system, the immunoglobulin family of proteins. The crystal structure of the CD8αα receptor in complex with the human MHC molecule HLA-A2 has demonstrated how the lg domain of CD8αα receptor binds the ligand;
c. membrane proximal stalk region (amino acids 116-160). This domain is thought to be an extended linker region allowing the CD8αα receptor to 'reach' from the surface of the T-cell over the top of the MHC complex to the α3 domain of this where it binds. The stalk region is glycosylated and thought to be inflexible. In the context of this invention it should be noted that the stalk region is assumed to play no part in MHC binding;
d. transmembrane domain (amino acids 161-188). The transmembrane domain anchors the CD8αα receptor in the cell membrane and is therefore not part of the soluble recombinant protein;
e. cytoplasmic domain (amino acids 189-214). The cytoplasmic tail of CD8 mediates a signalling function in T-cells through its association with p56^{*lck*} which is involved in the T cell activation cascade of phosphorylation events.

The invention encompasses two forms of the CD8 receptor, αα and *α*β (EMBL/GENBANK database accession numbers: CD8α, M27 161; CD8β, X13444). The two forms of the receptor are functionally equivalent and no significant differences in the effects of using one or the other for immune inhibition would be expected.

The essential feature of any soluble CD8 protein envisaged is that it contains a correctly folded lg domain since it is this region that constitutes the binding region for contacting MHC molecules and therefore is responsible for the modulating effect. Variants of the particular CD8 proteins which are the subject of the invention are envisaged and are listed as follows:
a. Variations of the C-terminal truncation point. It is possible that longer or shorter versions of the receptor could also be stable and functional. There is no general rule to predict where the optimal truncation point for a soluble version of a transmembrane protein is. In the case of CD8, the polypeptide could be between 1 and 15 amino acids longer or shorter. However, when shortened at the C terminus the molecule still retains a short fragment of the membrane-proximal stalk region. The soluble CD8 could even comprise the cytoplasmic domain, having just the transmembrane domain deleted. It is also envisaged that the C-terminus could be fused to peptides or protein domains, such as glutathione-S-transferase for purification purposes, as well known in the art.
b. Variations in the N-terminal truncation point. There is some uncertainty as to where exactly the signal sequence ends and the lg domain starts. The N-terminal truncation point could probably be varied somewhat, just like the C-terminal truncation point, without any influence on the functional effect of the protein. It is also envisaged that the N-terminus could be fused to peptides or protein domains, such as glutathione-S-transferase for purification purposes, as well known in the art.
c. Amino acid substitutions. A large number of conservative amino acid substitutions can presumably be introduced in the protein without causing any significant change in the binding between CD8 receptor and MHC.
d. Mutations designed to alter functionality. With the structure of the complex between CD8αα receptor and a MHC molecule, HLA-A2. (Gao *et al* 1997) it is possible to design mutations predicted to affect the interaction and therefore the immuno-inhibitory effect. The testing of two such mutations is described below, the substitution of residue 99 by a glutamic acid (designated ' 99E') and the double substitution of residues 54 and 55 by glutamic acids (designated '54,55E'). These mutations were designed as controls, i.e. the mutations were intended to abolish the binding of the CD8αα receptor to MHC. Mutant 99E has a residual effect while mutant 54, 55E shows no sign of inhibition (see following T cell lysis data contained below). Thus the design of mutations with the opposite effect, i.e. which increase the binding between CD8αα receptor and MHC molecules is also envisaged.

The examples that follow are more clearly described with reference to the figures, of which:

Figure 1b shows the amino acid sequence of a soluble recombinant human CD8α protein.

Figure 2 shows CD8α and truncated proteins expressed in *E.coli.* Schematic presentation of CD8α illustrating domain organization (top) and the extent of the truncated proteins expressed in bacteria (below): L = leader sequence, lg = immunoglobuiin domain, MP = membrane proximal domain, Tm = transmembrane domain, Cyt. = cytoplasmic domain. Numbers indicate amino acid positions of domain boundaries in relation to the mature protein and the endpoints of proteins expressed in *E*.*coli*. 'C' signifies the presence and positions of the cysteine residues at positions 143 and 160 involved in forming the interchain disulfide bonds in the CD8αα dimer. Expression vectors containing the codon information for the illustrated truncated proteins were constructed both with the original codon usage for human CD8α and with codon usage changes in the 5' end of the insert as described in the Examples.

Figure 19 shows the amino acid sequence of a soluble recombinant mouse CD8α protein.

The figures are discussed in detail in the Examples.

### EXAMPLES

### Example 1. High level expression of CD8αα homodimer in E. coli and crystallisation.

### MATERIALS AND METHODS

### Cloning of CD8α cDNA and Construction of E.coli Expression Vectors -

A CD8α encoding DNA fragment was obtained by the polymerase chain reaction (PCR) using cloned Pfu polymerase (Stratagene) and cytotoxic lymphocyte cDNA as template. The PCR reaction was performed with the sense primer: 
5'-GACTGAGTCGCGGCCGCTGCCACCATGGCCTTACCAGTGACCGCC
TTG-3' [SEQ ID NO: 1];
 and the antisense primer:

5'-TATTCGACTGGATCCTTATACGTATCTCGCCGAAAGGCTGGG-3'
[SEQ ID NO: 2].

The PCR product was restriction digested with Eag I and BamHI and cloned into pBluescript II KS- (Stratagene) to obtain plasmid BJ082. The presence of the full length CD8α coding sequence was verified by doublestranded dideoxy sequencing (USB 70754) using T7 polymerase (Pharmacia). For the construction of bacterial expression vectors five PCR products using BJ082 as template were generated which encode a methionine followed by the mature immunoglobulin like domain of CD8α. The PCR reactions were conducted with the sense primer 
5'-GGAATTCCATATGAGCCAGTTCCGGGTGTCGCCGCTGGATCG-3'
[SEQ ID NO: 3]
 and the antisense primers:
A: 
5'-CGCGGATCCCTATGCGCCCCCCGCTGGCCGGCTCGCCTCTGGGC
GCAGGGACAG-3' [SEQ ID NO: 4];
B: 
5'-CACCGCGAATTCGGATCCTAAGCGGGTCTACAAGCTTCGGGCTTC
GCTGGCAGGAAGACC-3' [SEQ ID NO: 5];
C: 
5'-CACCGCGAATTCGGATCCTAAGCGGGTCTACAAGCTTCTGGCGTC
GTGGTGGGCTTCGC-3' [SEQ ID NO: 6];
D: 
5'-CACCGCGAATTCGGATCCTAAGCGGGTCTACAAGCTTCGGGCTTC
GCTGGCAGGAAGACC-3' [SEQ ID NO: 7];
or E:

5'-GTGGCAAGCTTGGATCCTATGGCGTCGTGGTGGGCTTCGCTG-3'
[SEQ ID NO: 8].

These PCR products were restriction digested with Nde I and Bam HI and cloned in vector pGMT7 to obtain expression cassettes for residues A: 1-150 with the cysteine residue at position 143 mutated to serine, B: 1-146, C: 1-120/141-146, D: 1-116/141-146, and E: 1-120. After sequence verification the vectors were tested for expression (see below) but induction of CD8α protein expression from all vectors was only detectable by immunoblot analysis employing the monoclonal antibody OKT-8 (ATCC reference CRL8014). Five new PCR fragments encoding the corresponding proteins were subsequently generated using a modified sense primer, 
5'-GGAATTCCATATGAGTCAATTTCGTGTATCACCGCTGGATCG-3'
[SEQ ID NO: 9],
 which introduces six silent mutations in the coding region. These PCR fragments were restriction digested with *Nde* I and *Hind* III and cloned into the vector pGMT7 to obtain plasmids UG195 (A), UG231 (B), UG222 (C), pAÆ3 (D), and BJ112 (E).

### Construction of HLA-A2 Expression Vector-

A DNA fragment encoding the α1, α2, and α3 domains of HLA-A*0201 (HLA-A2), comprising residues 1-276, was obtained by PCR using B cell cDNA as template. The PCR reaction employed the sense primer: 
5'-ACATACCCATGGGCTCTCACTCCATGAGGTATTTC-3' [SEQ
ID NO: 10];
 and the antisense primer:

5'-ACATACAAGCTTACGGCTCCCATCTTAAGGTGAGGGGCTTGGG-3'
[SEQ ID NO: 11].

The PCR product was restriction digested with *Nco* I and *Hind* Ill and cloned in pET23d+ (Novagen) to obtain plasmid BJ075a in which the sequence of the HLA-A2 fragment was verified by dideoxy sequencing. The plasmid pHN1- β2m used for expression of the β2 microglobulin subunit was kindly provided by David N. Garboczi and Don C. Wiley, Harvard Medical School.

### Expression of CD8α in E.coli -

T7-CD8α expression vectors were transformed into the *E.coli* strain BL21DE3pLysS (Novagen) and from single colonies cultures were grown in TYP medium containing 100 µg/ml Ampicillin. Expression from the T7 promoter was induced at approximately OD600 = 0.4 by the addition of IPTG to a final concentration of 0.5 mM and the time-course of induction initially followed for up to eight hours. No increase in protein yields were observed with any constructs after 4 hours following induction by IPTG.

### Large Scale Preparation and Solubilization of CD8α from Bacterial Inclusion Bodies -

Bacterial cells were recovered by centrifugation and resuspended in Lysis Buffer (20 ml per liter original bacterial culture) containing 10 mM EDTA, 2 mM DTT, 10mM Tris-HCI (pH 8.0), 150 mM NaCl, 10% v/v Glycerol, 200 mg/l Lysozyme, 1.0mM PMSF. The solution was freeze-thawed and sonicated (Misonix W38S) for a total of 5 min. After mixing with an equal volume of Wash Buffer (0.5% v/v Triton X-100, 10 mM EDTA, 50 mM Tris-HCI (pH 8.0), 100 mM NaCl, 10 mM DTT, 0.1% v/v Sodium azide) the solution was left on ice for a further 30 min. Inclusion bodies were pelleted by centrifugation at 15,000 rpm in a Beckman JA-20 rotor for 10 min., then resuspended in Wash Buffer (20 ml per liter original culture) using a teflon homogeniser. The recovery and wash steps were repeated four times in total. Following the last spin the pellet was solubilized in Urea Buffer made up as 8 M Urea, 20 mM Tris-HCl (pH 8.0), 10 mM EDTA, 10% v/v Glycerol, 500 mM NaCl, 10 mM DTT and end-over-end mixed overnight at 4°C. Debris was cleared from the inclusion body suspension by centrifugation as described above for 30 min. and the cleared suspension stored at -70°C.

### Refolding of the CD8αα Receptor -

The CD8α protein (at 10 mg/ml) in 2 ml Urea Buffer was added to 15 ml Guanidine Buffer (6 M Guanidine hydrochloride, 50 mM Tris-HCI (pH 8.0), 10 mM EDTA, 100 mM NaCl, 10 mM DTT) and allowed to equilibrate at room temperature for 30 min., then added to 1 litre Refolding Buffer made up as 200 mM Tris-HCI (pH 8.0), 10 mM EDTA, 1 M L-arginine, 0.1 mM PMSF, 6.5 mM Cysteamine and 3.7 mM Cystamine. Two more pulses of 20 mg protein were added to the refolding mixture at 24 hour intervals and the solution stirred at 4°C for 72 hours in total.

### Refolding of HLA-A2/pol -

HLA-A2 heavy chain, β2m and peptide (ILKEPVHGV [SEQ ID NO: 12], synthesized by Oxford Centre for Molecular Sciences and corresponding to residues 309-317 of HIV-1 reverse transcriptase) were refolded essentially as described with the modification that Guanidine Buffer was added to the protein before injecting this into Refolding Buffer as described above for folding of CD8α (David N. Garboczi, personal communication).

### Purification of Folded CD8αα Receptor -

The refolding mixture was cleared by centrifugation for 30 min. at 4,000 rpm in a Beckman J-6B, after which the supernatant was concentrated to approximately 200 ml in an Amicon 2000 Stirred Cell using a Diaflow ultrafiltration membrane with 10 kD pore exclusion size. Following this the sample was centrifuged for 30 min. at 15,000 rpm in a Beckman JA-20 rotor, then further concentrated to 10 ml in an Amicon 8400 Stirred Cell. Prior to a two-step purification procedure involving gel filtration and cation exchange the concentrated sample was filtered through a 0.22 µm sterile filter. Subsequent preparations of CD8α have indicated that the centrifugation and filtration steps introduced to clear the protein solution between refolding and concentrating are essential for maintaining correctly folded receptor in solution. The gel filtration step was performed on a Pharmacia Superdex G-75 column equilibrated in 20 mM Tris-HCI (pH 8.0), 150 mM NaCl. Fractions containing CD8αα homodimer were collected and buffer exchanged into 50 mM MES (pH 6.5) using an Amicon Centriprep-10. The ion exchange step was performed on a Pharmacia Mono-S column from which the protein was eluted with a gradient of 0-1 M NaCl in 50 mM MES (pH 6.5).

### Biochemical and Physical Analysis of Purified Proteins -

Protein concentrations of the folded and purified proteins were determined by Bradford Assay (BioRad). The identity of the folded CD8α was confirmed by Edman sequencing of the N-terminus. Mass spectroscopic analysis of CD8αα was performed using a VG Instruments triple-quadropole atmospheric mass spectrometer fitted with an electrospray ionisation interface. Protein samples of 10 µg in 20 µl 1:1 v/v acetonitrile/water, acidified by addition of formic acid to a final concentration of 1% v/v, were injected at a flow rate of 20 µl/min. The mass spectrometer was calibrated using horse heart myoglobin (Mr 16951.48).

### Crystallisation of the CD8αα/HLA-A2 Complex -

1 µl protein solution containing CD8αα at 10 mg/ml and HLA-A2/pol at 20 mg/ml were mixed with 1 µl reservoir solution from Crystal Screen kits I and II (Hampton Research). Crystallization trials were performed by vapour diffusion from sitting drops on microbridges at room temperature.

### RESULTS

### Mutations that Decrease the Tendency for Secondary mRNA Structures in the 5' end of the CD8α Gene Greatly Increase the Expression Potential in E. coli -

To construct CD8α expression vectors for use in a range of expression systems a DNA fragment constituting the entire open reading frame was first generated by the polymerase chain reaction (PCR) using cDNA prepared from a cytotoxic T-lymphocyte cell line as template. A plasmid containing this fragment was then used as template in a second generation of PCR reactions to synthesize five CD8α cDNA fragments which were inserted in plasmids containing the T7 promoter. These constructs all encode polypeptide chains in which an initiation codon is followed by the mature CD8α N-terminal region, comprising the entire immunoglobulin domain but truncated at different points C-terminally of this (Fig. 2). Constructs with equivalent C-terminal truncations but incorporating the CD8α signal sequence were tested for expression from a baculovirus promoter in insect cells.

Previously, expression of human CD8α in insect and CHO cells has indicated that disulfide bonding of the dimer through Cys143 is critical for the production of a secreted receptor. In contrast, Cys160 which is also involved in covalently linking the membrane-bound receptor was found to prevent expression of soluble CD8αα. In accordance with this we found that in insect cells CD8α 1-146, 1-120/141-146 and 1-116/141-146 could be expressed as secreted dimers (data not shown). The requirement for the Cys143 residue for secretion of CD8αα dimer from insect cells indicates that this residue is essential either for the folding of the protein or for its stability during intracellular transport. These possibilities can not at present be distinguished and since cysteine residues pose a potential problem when refolding protein *in vitro* constructs incorporating or omitting Cys143 were tested in bacteria (Fig. 2).

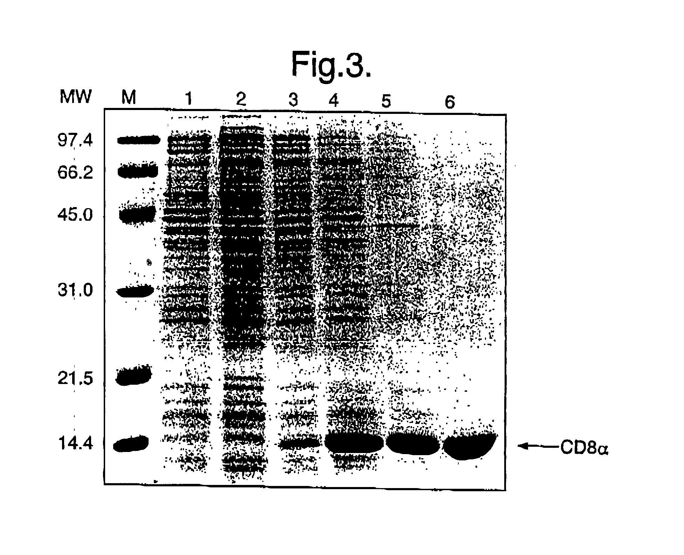

From plasmids containing inserts with the original human CD8α codon usage no induction of expression could be detected in *E.coli* as assessed by comparison of whole cell extracts after gel electrophoresis and coomassie staining (Fig. 3, lanes 1-2). In contrast, expression was readily detectable when six codons in the CD8α gene were altered by silent mutations to make the 5' end of the gene more A/T rich (Fig. 3, lanes 3-4). It has previously been reported that such changes, as would be expected with a lower G/C content and therefore weaker base-pairing potential, decrease the tendency for hairpin formation in the 5' end of the CD8α mRNA. It appears likely, therefore, that secondary structure formation in the original sequence has a severe adverse effect on the potential for expression in *E. coli*. Expression from the mutated constructs are estimated to yield in excess of 100 mg per litre, an increment of at least two orders of magnitude over the expression level obtained with the unmutated sequence.

### Large-scale Preparation, Initial Purification and Solubilization of Bacterially Expressed Proteins -

Analysis of cellular fractions from lysed bacteria expressing CD8α showed that virtually all the protein is deposited in inclusion bodies (Fig. 3) as has previously been observed with bacterially expressed MHC heavy chain and β2m polypeptides. Purification of the CD8α inclusion bodies by washing yielded protein estimated to be approximately 90% pure (Fig. 3, lane 5). Similar inclusion body purity was obtained with HLA-A2 and β2m (not shown). CD8α inclusion bodies were not soluble in the pH 6.5 buffer used for the MHC chains but adjustment of pH to 8.0, high salt and the addition of 10% Glycerol made it readily soluble.

### Refolding and Quality Analysis of Soluble CD8αα Receptor -

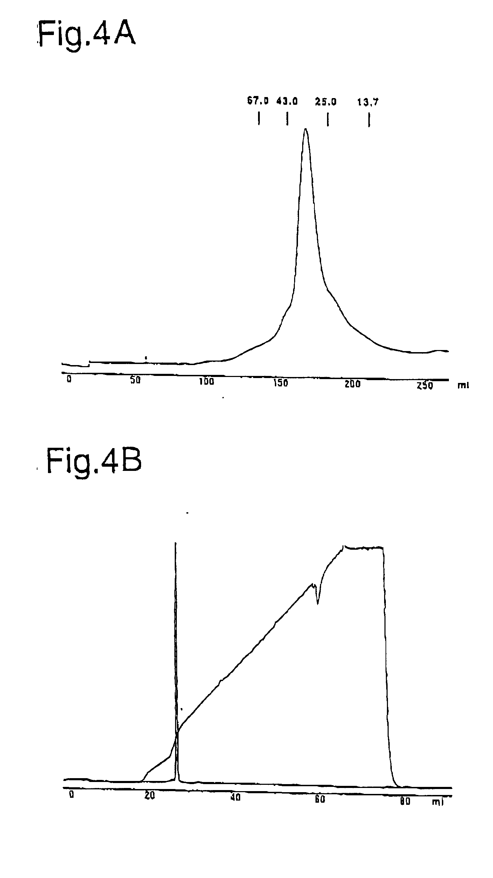

The resolubilized CD8α polypeptide in reducing and denaturing buffer was diluted into conditions permitting formation of native protein conformation. After allowing folding to proceed for 48 hours soluble protein was concentrated for analysis and purification by gel filtration. Four of the polypeptides expressed and illustrated in Fig. 2 produced mainly aggregate peaks of high molecular weight (data not shown). In contrast to this the elution profile of the 1-120 polypeptide shows one major peak of approximately 30 kDa as expected for the CD8αα dimer (Fig. 4A). In this elution profile no indication of aggregates is evident. The CD8αα dimer was further purified by cation exchange on a Mono-S column eluting as a single peak at approximately 175 mM NaCl (Fig 4B). Gel analysis of this peak shows only one band at the expected position for CD8α (Fig. 3, lane 6).

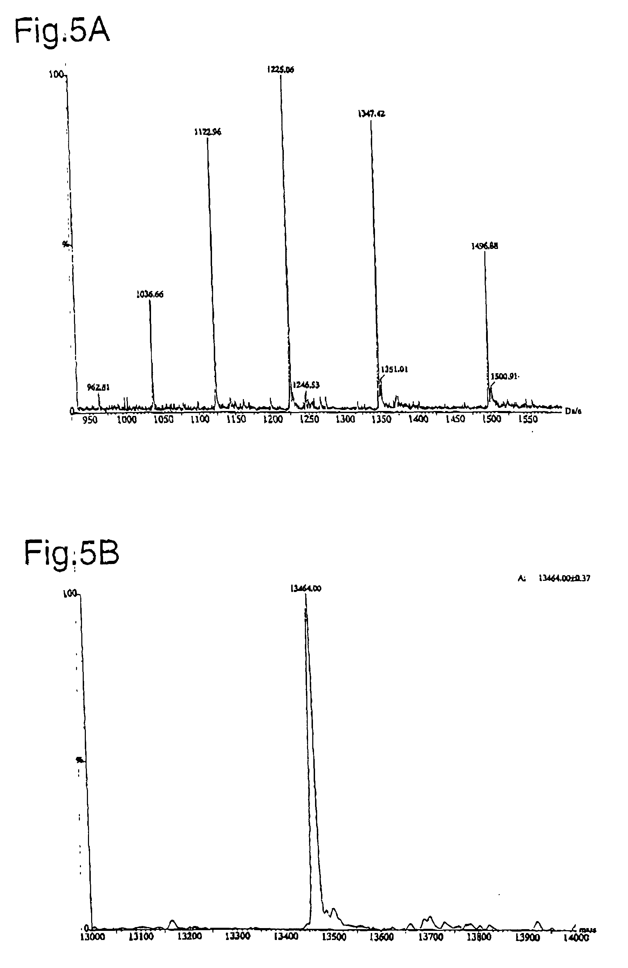

The identity of the purified CD8α was verified by two methods. Edman sequencing produced a run of ten amino acids corresponding to the mature N-terminus of CD8α showing that the introduced methionine residue had been cleaved off in *E.coli.* Electrospray mass spectrometry confirmed the molecular weight of CD8α (13,464.0) as very close to the theoretical value of 13,463.2 Dalton for residues 1-120 excluding the initiating methionine (Fig. 5). A smaller peak of slightly higher molecular weight is likely to correspond to protein that has not had the methionine residue cleaved off (Fig. 5).

### Co-refolding and Soluble Complex Formation between CD8αα Dimer and HLA-A2/pol -

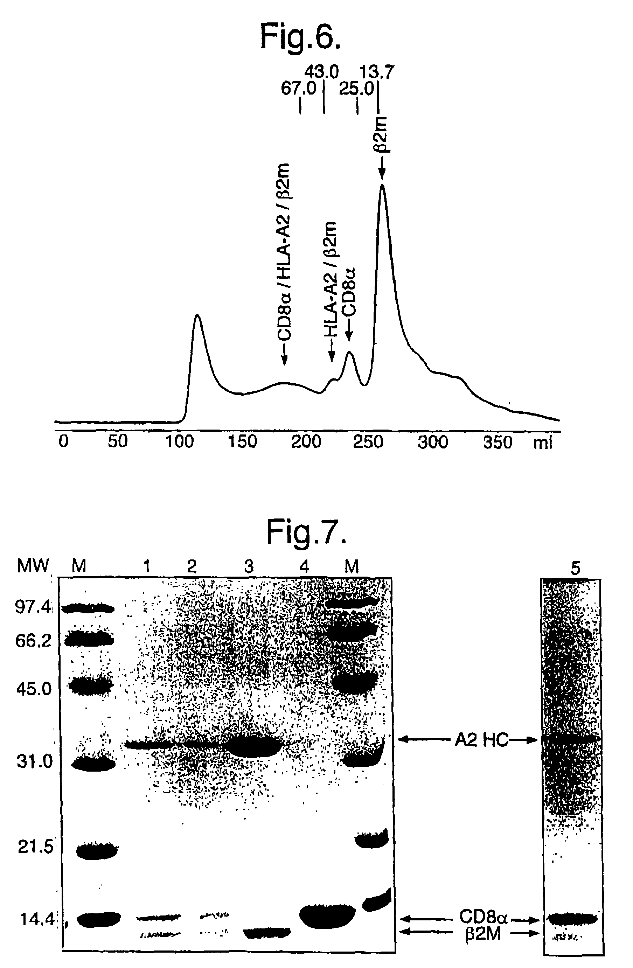

To test whether the efficiency of refolding of the component molecules could be increased by complex formation a co-refolding experiment was performed. Gel filtration analysis of the concentrated protein reveals that the folding of both CD8αα and HLA-A2 are impaired and that more aggregates are formed under these conditions than during individual refolding (Fig. 6). A peak is detectable, however, at the expected position corresponding to approximately 75 kDa for the CD8αα /HLA-A2 complex which is not present in either the CD8α refolding (Fig. 4A) or in the HLA-A2 refolding (not shown). Analysis of the protein in this peak shows that the components expected in the complex are present in amounts corresponding to one CD8αα dimer to one HLA-A2 (Fig. 7, lane 1 and 5) indicating that stable complex formation can take place in solution.

### Crystallization of CD8αα in Complex with HLA-A2/pol -

Crystallization trials, performed with a 2:1 mixture of separately refolded HLA-A2 and CD8αα, yielded visible crystals under two buffer conditions, 12% PEG 20,000, 100 mM MES pH 6.5 and 15% PEG 6000, 50 mM MES pH 6.0, after approximately seven days at room temperature. One crystal was later diffracted at 2.7 Å resolution. To analyze the composition of the crystal a fragment was washed, resolubilised and analysed by gel electrophoresis (Fig. 7, lane 2). The protein bands present in this sample correspond exactly to those from the individual HLA-A2 and CD8α (Fig 7, lanes 3 and 4, respectively). Furthermore, the ratio of the CD8α to the heavy chain and β2m bands are identical to those found in the sample from the co-refolding peak thought to correspond to the receptor -ligand complex (Fig. 7, lane 1).

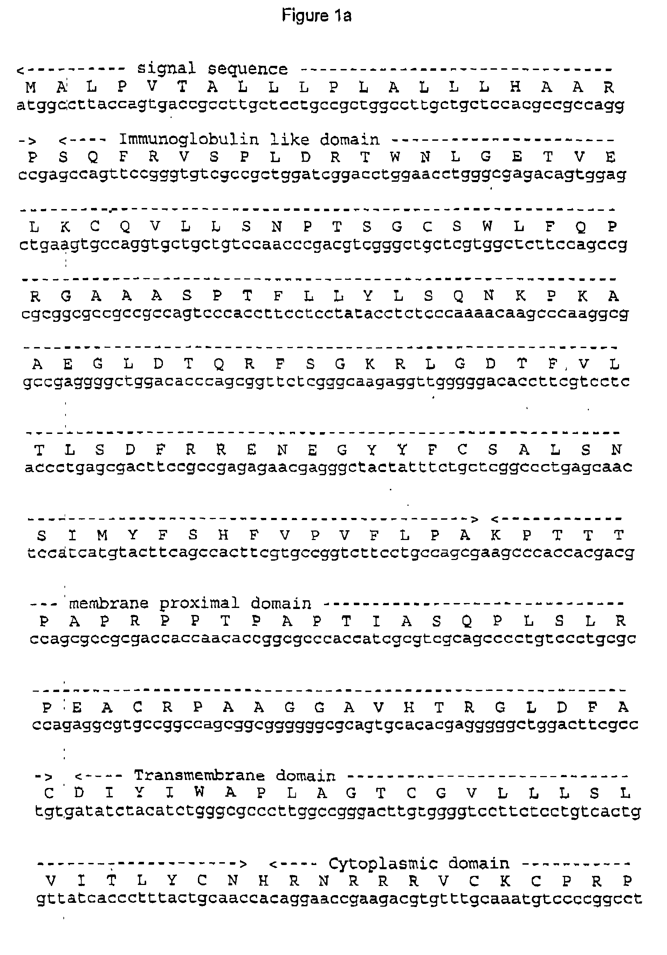

### Legends for Figures 1 and 3 to 7

Figure 1a shows cDNA sequence (bottom) and protein sequence (top, one-letter code) of human CD8α. The extent of the signal peptide, lg-like domain, membrane proximal domain, transmembrane domain and cytoplasmic domain are indicated.

Figure 1b shows DNA sequence (bottom) and protein sequence (top, one-letter code) of the expressed fragment of human CD8α. The extent of the lg-like domain and membrane proximal is indicated. Bases added on to the DNA sequence in order to encode a translation initiation codon or to enhance expression in *E.coli* are indicated in lower case letters.

Figure 3 shows expression and purification of CD8α. Approximately 10µg protein samples were analyzed by separation on a 15% SDS-PAGE gel. M = protein size marker; lane 1 = whole lysate of uninduced BL21 cells containing the expression plasmid for residues 1-120 of CD8α with original codon usage; lane 2 = as lane 1 but induced with 0.5 mM IPTG for 4 hours; lane 3 = lysate of uninduced BL21 cells containing CD8α expression plasmid with altered codon usage at the 5' end; lane 4 = as lane 3 but induced with 0.5 mM IPTG for 4 hours; lane 5 = purified inclusion body fraction from cells as in lane 4; lane 6 = refolded and purified by gel filtration and ion exchange. The molecular weight of the size markers and the expected position of CD8α are indicated.

Figure 4 shows purification of CD8α. *A*, Superdex G-75 gel filtration profile of concentrated CD8α refolding. Numbers over the profile indicate the elution points of molecular weight standards. *B*, NaCl elution profile from Mono-S column of the main peak from the gel filtration.

Figure 5 shows electrospray mass spectrometry analysis of soluble CD8αα receptor. *A*, electrospray mass spectrum of CD8αα showing mass-to charge ratio peaks after proton bombardment; *B*, deconvolution of the spectrum shown in *A* indicating the molecular weight of the single dominant peak.

Figure 6 shows co-refolding gel filtration profile of CD8α and HLA-A2. Gel filtration was performed on a Superdex G-200 column. Numbers over the profile indicate the elution points of molecular weight standards. The protein components of the obtained peaks are indicated over the arrows.

Figure 7 shows SDS-PAGE analysis of co-refolded and co-crystallized CD8αα and HLA-A2. Lanes 1-4 and M were stained with Coomassie Blue, lane 5 was silver stained. Lane 1 and 5: co-refolded CD8α, HLA-A2, β2m and peptide from peak indicated in Figure 5; lane 2: resolubilized crystal; lane 3: HLA-A2; lane 4: CD8αα, M: protein size markers as indicated. The bands corresponding to A2 heavy chain (A2 HC), β2m and CD8α are indicated with arrows.

### DISCUSSION

### Expression of CD8α

For expression of CD8α we found the yields obtained from insect cells insufficient for large-scale purification and therefore focussed on expression in *E.coli*. In bacteria three parameters generally appear to be the main determinants of the expression level which can be achieved for a foreign protein. These are the tendency for secondary structure in the mRNA, the degree to which the codon usage conforms to bacterial preference, and the toxicity for the host of the expressed protein. In the case of CD8α, six silent codon changes, designed to decrease base-pairing potential close to the N-terminus, increased the expression level by more than two orders of magnitude to a level constituting approximately 30% of the total protein. The high expression levels and the ease with which the protein can be purified from inclusion bodies made bacterial expression the preferred choice for further studies.

### Refolding of CD8α

The refolding efficiency of CD8α was found to depend on the buffer conditions used to solubilise the protein from inclusion bodies. In particular the maintenance of a high redox potential, high ion strength and the addition of strongly denaturing agents before refolding dramatically improves the yield of the CD8αα dimer. Analysis of the folded receptor preparation by gel filtration shows virtually no presence of aggregates and after cation exchange the preparation appears to be completely homogeneous and stable for several months at 4°C.

### Analysis of the CD8αα-HLA-A2 Complex Formed in Solution and by Crystallization

The estimated molar ratios of the polypeptide bands representing HLA-A2 assembly and CD8αα are identical in the complex formed in solution and in the crystal but the stoichiometry indicated by the band intensities is somewhat unexpected. Because CD8 has been detected on the T cell surface as a dimer it has been speculated that CD8 would bind two MHC molecules and that a bivalent stoichiometry could be involved in T cell-target cell ligation and T cell activation. However, the gel analysis of the complexes, taking into consideration that CD8α stains weaker with coomassie than does β2m, are more indicative of a 1:1 HLA-A2:CD8αα ratio and this has been confirmed by the crystal structure. Based on the crystal structure steric considerations rule out that more than one CD8αα dimer can bind the MHC.

Recent studies involving cells or soluble murine CD8 and TCR have indicated that a mechanism for cooperative binding to class I MHC may exist for the two receptors. Comparison of the crystal structures of human CD8αα/HLA and TCR/HLA indicate that both receptors introduce a shift in the α3 domain of the HLA heavy chain which could be associated with an energetic penalty upon binding. Further studies on complexes of MHC Class I with human CD8 and TCR should clarify whether the structural shift introduced in the HLA heavy chain constitutes a mechanism for cooperative binding of these receptors.

### Example 2. Immune-inhibitory effect of the CD8αα receptor.

In a standard cytotoxic T cell assay suitable target cells, i.e. cells which express the relevant restrictive MHC, are first incubated with peptide (1 hour) and radioactive chromium. After washing the target cells are exposed to T cells. The T cell, through T cell receptor (TCR) and CD8, recognises the complex of the peptide and MHC on the target cell surface and, if the ligand is appropriate and present in sufficient concentration, the T cell will lyse the target cell. Lysis is assessed by measuring the release of radioactive chromium.

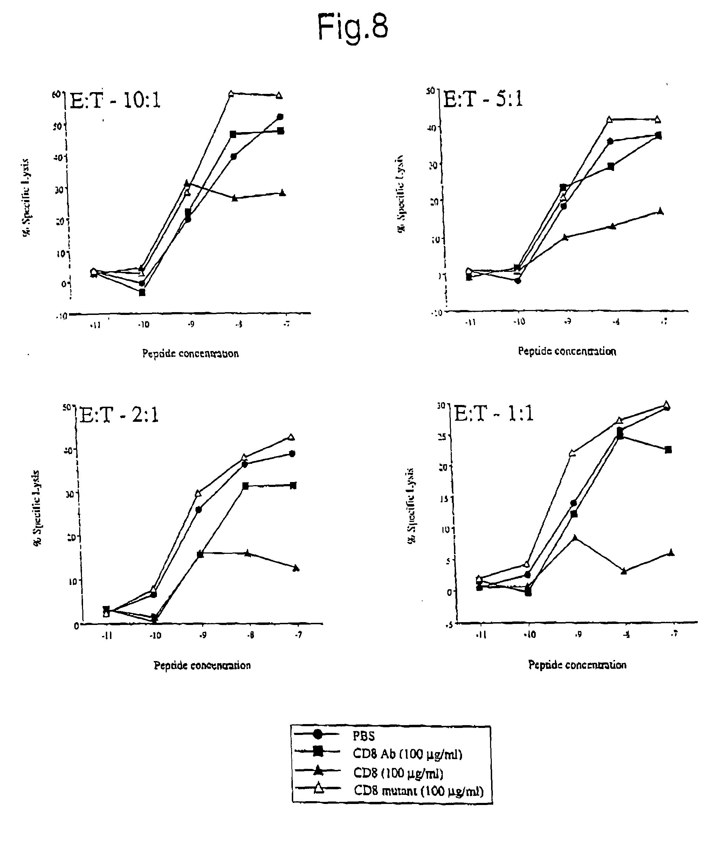

Figure 8 shows the results of a series of experiments conducted as follows:

A human T cell line (enriched and selected), recognising the HIV1 'gag' peptide (SLYNTVATL [SEQ ID NO: 13]) restricted by HLA-A2, was used. A constant number of target cells, 5000, '868' B cells expressing HLA-A*0201, were used and the number of T cells was varied. 'E:T' represents a ratio of numbers of T cells to target cells. Four E:T ratios were tested. The peptide concentration was varied from 10⁻¹¹ to 10⁻⁷ M (horizontal axis on the plots). (The units of peptide concentration are log₁₀M unless otherwise marked). Four sets of experiments probing the CD8 effect were conducted, these are marked by the legend on the chart as:
'PBS' - incubation with cell medium alone (phosphate buffered saline), as a control;
'CD8 Ab' - incubation with an anti-CD8 monoclonal antibody (100 µg/ml), OKT8, which should have some inhibitory effect by binding to CD8 on the T cell surface and blocking the interaction between this and HLA-A2;
'CD8' - incubation in the presence of soluble CD8 receptor, at 100 µg/ml;
'CD8 mutant' - incubation in the presence of soluble CD8 receptor with the 54-55E double mutation at 100 µg/ml.

The assay components for these experiments were:
18 µl 10X peptide
18 µl PBS or PBS with 1mg/ml CD8αα or CD8 OKT8 Ab
50 µl Targets (5,000 868 B cells)
100 µl containing 5,000 -50,000 CTL.

The graphs of figure 8 show the results collected after 2 hours incubation.

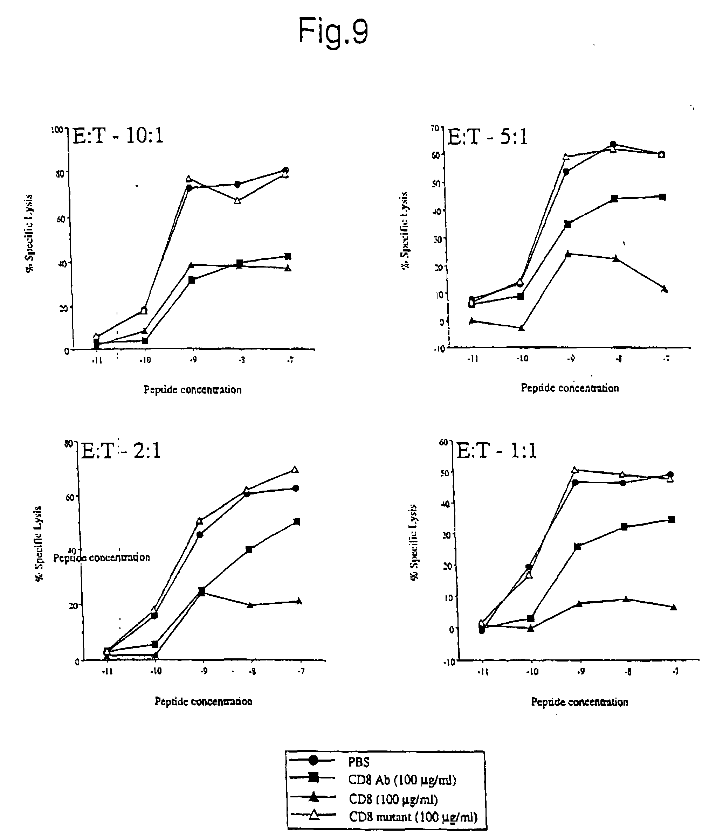

Figure 9 shows data from the same experiments as Figure 8 except that the data were collected after 6 hours incubation.

The data in figures 8 and 9 demonstrate:
- that more target cells are killed at high peptide concentration which is as expected. At low concentration the density of specific target molecules on the target cell surface is too low to activate the T cells.
- CD8 antibody which blocks CD8 on the T cells has some inhibitory effect compared to PBS (control).
- Soluble CD8, which must be assumed to bind the MHC on the target cells, has a clear inhibitory effect, even better than that of the CD8 antibody.
- The effect of the soluble CD8 is specifically related to its binding to MHC as the mutant which has two critical amino acids substituted has no inhibitory effect.

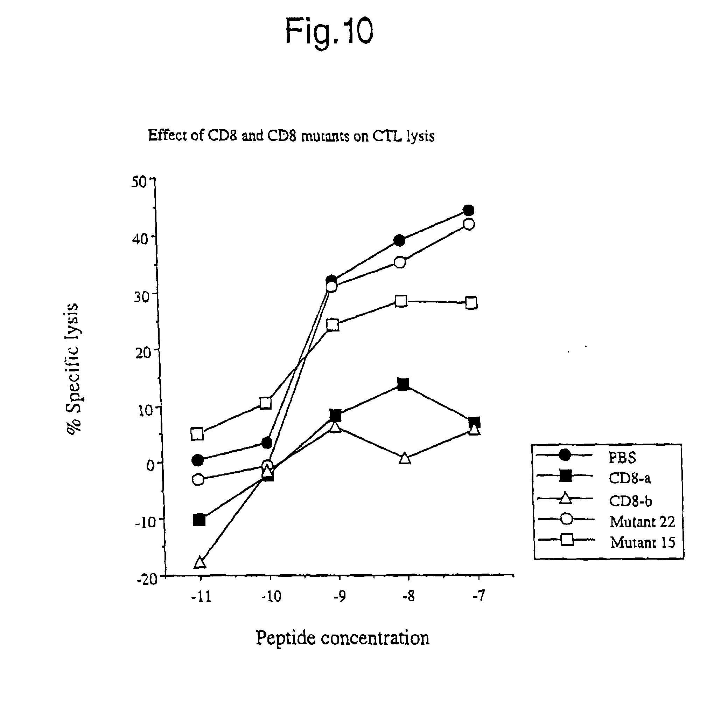

Figure 10 shows the results of a series of experiments conducted as follows. The same human T cell line recognising the HIV1 'gag' peptide restricted by HLA-A2 was used as in the experiments of Figures 8 and 9. A constant number of target cells and T cells, 5000 of each were used. The peptide concentration was varied from 10⁻¹¹ to 10⁻⁷ M (horizontal axis on the plots).

Five sets of experiments probing the CD8 effect were conducted:
'PBS' - incubation with cell medium as a control;
'CD8 A' - incubation in the presence of one preparation of soluble CD8 receptor at 100 µg/ml;
'CD8 B' - incubation in the presence of a second preparation of soluble CD8 receptor at 100 µg/ml;
'Mutant 22' - incubation in the presence of soluble CD8 receptor with the 54-55E double mutation at 100 µg/ml;
'Mutant 15' - incubation in the presence of soluble CD8 receptor with the 99E mutation at 100 µg/ml.

Results were collected after 2 hours incubation.

The data in figure 10 extend the conclusions drawn from data in figures 8 and 9, demonstrating similar effects of two preparations of soluble CD8 compared to two mutant CD8s. The E:T ratio was 1:1 and a 2 hour assay was performed.

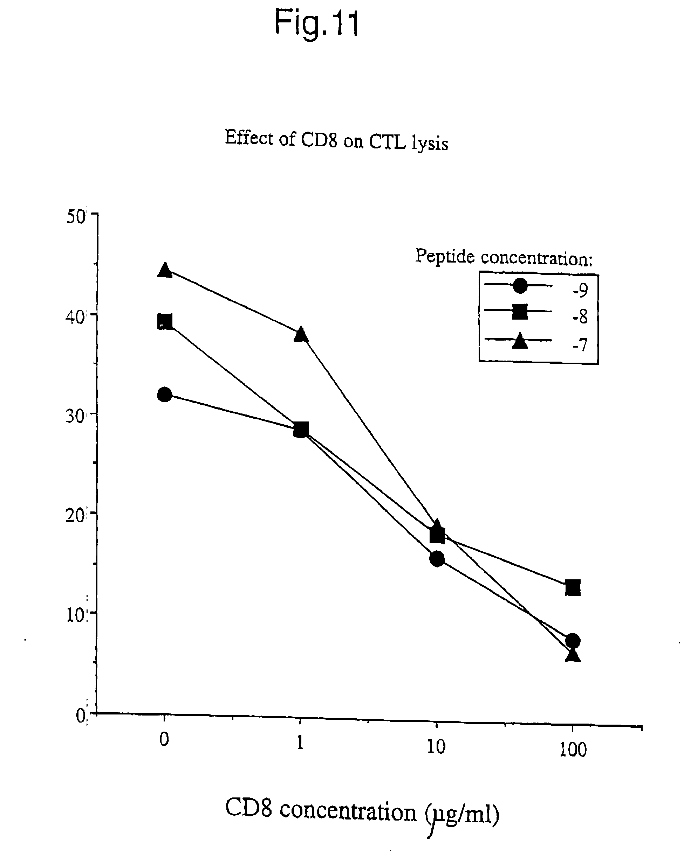

Figure 11 shows experiments performed as described above except that the concentration of soluble CD8 was varied from 0-100 µg/ml. The three traces correspond to peptide concentrations of 10⁻⁹, 10⁻⁸ and 10⁻⁷ M. The E:T ratio was 1:1 and a 2 hour assay was performed.

The data in figure 11 demonstrate that the CD8 inhibition of cell lysis is titratable.

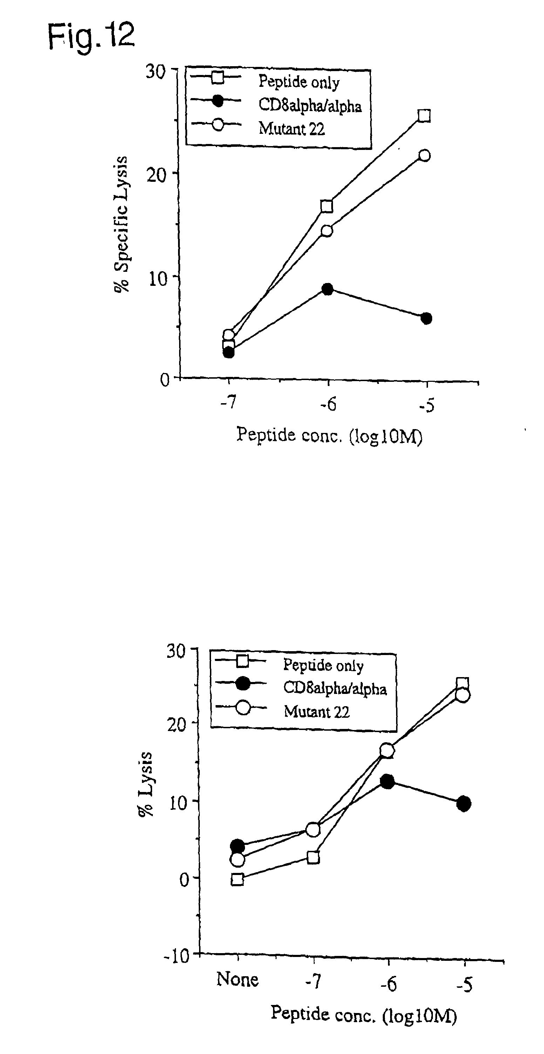

Figure 12 shows data from an experiment similar to those outlined above except that a T cell clone specific for HIV1 peptide 'pol' (VIYQYMDDL [SEQ ID NO: 14]) restricted by HLA-A2 was used. The targets were 868 B cells. E:T ratio was 5/1, peptide concentration varied between 10⁻⁷ and 10⁻⁵ M. 'Lysis' shows the absolute readout, 'specific lysis' shows results corrected for background.

The data in figure 12 demonstrate that the inhibitory effect of CD8 is also observed with a T cell clone.

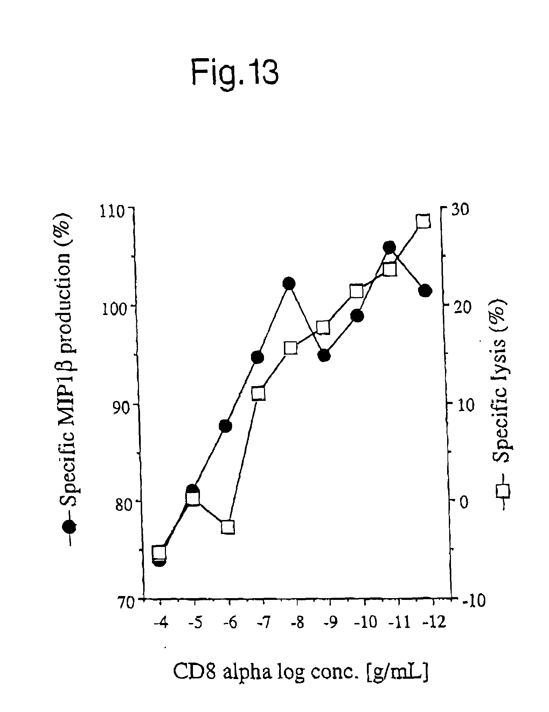

Figure 13 is another titration of CD8 inhibition showing both target cell lysis and T cell stimulation as assessed by MIP1β production. MIP1β is a T-cell activation marker which is produced by T cells in amount proportional to their activation state. This demonstrates that the inhibitory effect is correlated to an inhibition of T cell activation. For the lysis experiment, E:T ratio was 8:1; peptide concentration was 10 µM; no peptide control contained 100 µg/ml CD8αα; subtracted baseline is no peptide/no CD8αα negative control. For the M1P experiment 10⁵ cells per well were used; 10 µM peptide was used in assay of total volume 186 µl.

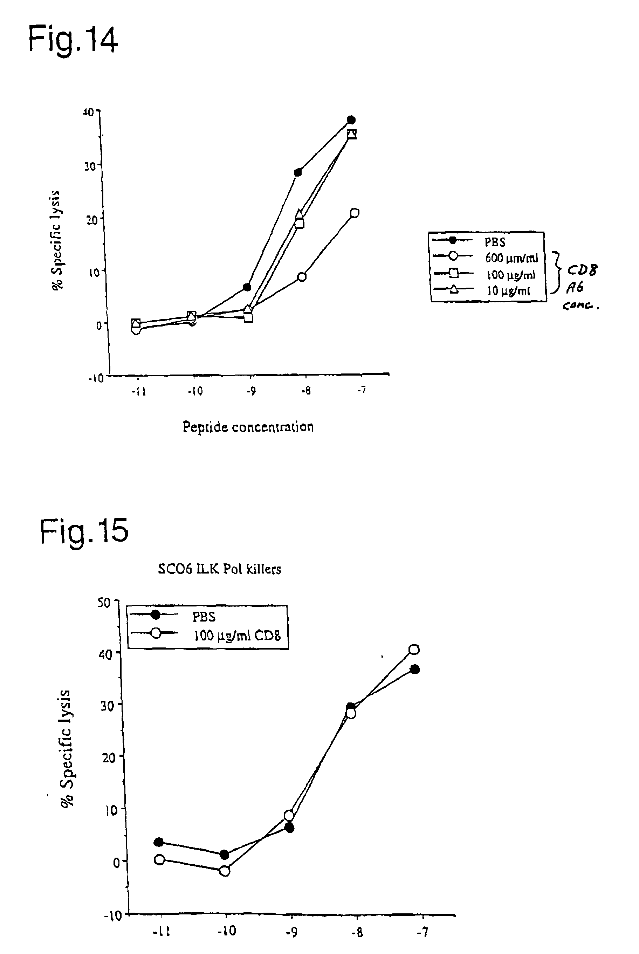

Figure 14 shows data from experiments involving a rare T cell resplonse that is independent of CD8. The experiment is run similarly to those previously described except that a different sample of cytotoxic T lymphocytes were incubated in a killing assay with '868' B cells at a E:T ratio of 5:1 and with varying concentrations of OKT8 CD8 antibody. The legend shows the data collected after 30 min:
'PBS' incubation with cell medium as a control
'600/100/10 µg/ml' - concentration of OKT8 CD8 antibody used in each incubation dataset

The data in figure 14 demonstrate that the CTL response for this particular sample of T cells cannot be inhibited by CD8 antibody except at extremely high concentrations, at which any protein would inhibit due to toxic effects. Thus the CTL response for this particular cell type is CD8 independent.

This experiment used a HLA-A2 Pol-restricted CTL line specific for the peptide ILKEPVHGV [SEQ ID NO: 12]. This cell line is CD8- independent as defined by the lack of inhibition of target lysis when preincubated with anti-CD8 antibody.

Figure 15 shows data from a similar experiment with the same cells incubated with soluble CD8 receptor at a range of peptide concentrations under similar conditions i.e. E:T 5:1, '868' B cells used as targets. The legend corresponds to:
'PBS' incubation with cell medium as a control
'CD8' incubation with soluble CD8αα receptor at 100 µg/ml.

The data in figure 15 demonstrate that the CTL response for this cell sample is not inhibited by soluble CD8.

Thus the data in figures 14 and 15, taken together, demonstrate that the soluble CD8 mediated inhibition observed with the CTL responses examined in figures 8 to 13 is not due to an unspecific effect.

### Example 3. Effect of sCD8αα on early protein tyrosine phosphorylation events in CD8- dependent and independent CTL

HLA matched, antigen pulsed, Epstein-Barr virus transformed, B cells were used as antigen presenting cells (APC). APC were incubated with soluble sCD8αα for 5 minutes prior to addition of CTL at an E:T ratio of 10:1. After incubating for 10 minutes at 37°C cells were pelleted and lysed in lysis buffer (Purboo *et al* 1998). Lysate was cleared by centrifuging at 13,000 rpm in a benchtop microfuge and was separated by SDS-PAGE. Gels were western blotted onto nitrocellulose membrane and the membrane subsequently probed with an anti-phosphotyrosine mAb. Detection of the primary antibody was by horseradish peroxidase (HRP) linked secondary antibody and enhanced chemiluminescence (ECL).

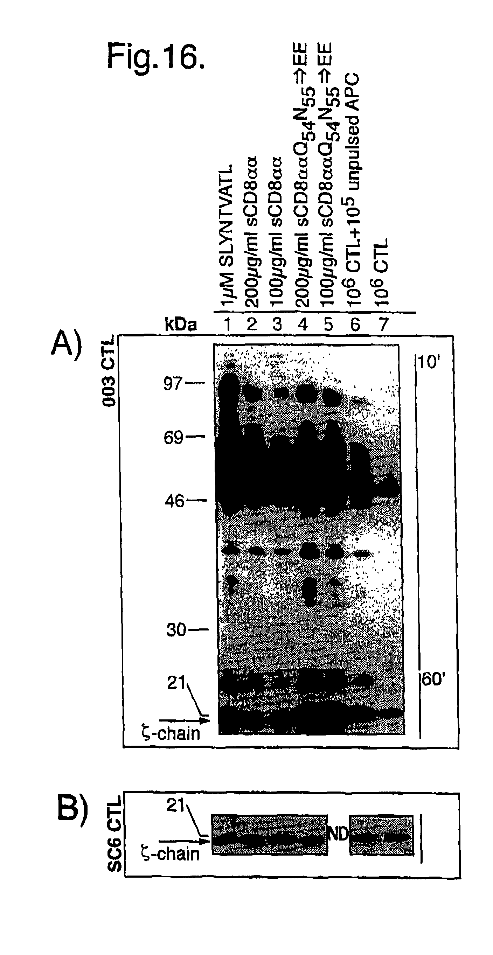

Results are shown in Figure 16. Anti-phosphotyrosine immunoblots of cell lysates from (**A**) 003 HLA-A2/Gag CTL clone (CD8-dependent) and from (**B**) SC6 HLA-A2/Pol CTL line (CD8-independent). Antigen pulsed B cells (10⁵) were presented to CTL (10⁶) either in the absence of sCD8αα (lane 1), in the presence of 200 or 100 µg/ml sCD8αα (lanes 2 and 3, respectively), or in the presence of 200 or 100 µg/ml of the CD8α Glu₅₄/Glu₅₅ mutant (lanes 4 and 5, respectively). Resting CTL (lane 7) and CTL presented with unpulsed B cells (lane 6) are also shown. To allow assessment of both overall protein tyrosine phosphorylation and, specifically, ζ-chain phosphorylation the upper and lower parts of the immunoblot were exposed for different times as indicated. Only ζ-chain tyrosine phosphorylation is shown for the CD8-independent CTL (**B**).

This experiment allowed evaluation of the intracellular effects on T cell activation (Fig. 16). In the presence of sCD8αα, protein tyrosine phosphorylation in the 003 Gag/HLA-A2 restricted CTL clone was not induced in response to the index peptide, resulting only in an activation state similar to that observed in response to B cells not pulsed with agonist peptide (Fig. 16A, lanes 2, 3 and 6). Thus, only peptide specific protein tyrosine phosphorylation is affected and the inhibitory effect of the sCD8αα therefore appears to be limited to signalling through the TCR/CD3 complex. The inhibition of CD3 ζ chain tyrosine phosphorylation by sCD8αα indicates that T cell activation is obstructed at the earliest stage of the signalling cascade, consistent with inhibition of p56^{*ick*} activation. No inhibitory effect on phosphorylation was observed in the presence of the Glu₅₄/Glu₅₅ mutant (Fig. 16A, lanes 4 and 5). In contrast to the observations involving CD8-dependent CTLs, no effect of either sCD8αα protein could be observed on CD3 ζ chain phosphorylation (Fig 16B), or on overall activation (not shown), in the SC6 Pol/HLA-A2 restricted, and CD8-independent, CTL line.

These results show that soluble CD8 receptor, through its specificity for MHC molecules, can function as a potent inhibitor of CD8-dependent CTL activation. Most striking is the observation that the sCD8αα completely abolishes early signalling events through the TCR/CD3 complex. This indicates that the soluble protein, at the employed concentrations, binds at a steady-state level which blocks the majority of CD8-MHC interaction sites, or that obstruction of a subset of MHC molecules for CD8 contacts, but not for TCR contacts, can negate signalling.

### Example 4. Expression and refolding of CD8αα with mutation of Cysteine 33 to Serine

In general, the factor which influences protein folding *in vitro* most is the number of cysteine residues in the polypeptide. The higher the number of cysteine residues, the more combinations are possible for the formation of disulfide bridges, both in the same polypeptide (intrachain disulfide bridges) and between two or more polypeptide chains (interchain disulfide bridges). Therefore, mutation of cysteine residues which are not paired in the native protein conformation is almost invariably beneficial to the yields which are obtained when refolding *in vitro.* Most frequently such residues are mutated to serine or alanine residues.

In CD8αα, the cysteine residue at position 33 of the mature protein is unpaired in the native conformation (Gao *et al*., 1997; Leahy *et al*., 1992). This residue has therefore, in order to increase the yields of CD8αα which can be obtained through *in vitro* folding, been mutated to a serine residue using the following primers in a PCR mutagenesis reaction (Stratagene): 
5' - CTG TCC AAC CCG ACG TCG GGC AGC TCG TGG CTC TTC CAG
CCG - 3' [SEQ ID NO: 15]
 and

5' - CGGCTG GAA GAG CCA CGA GCT GCC CGA CGT CGG GTT
GGA CAG - 3' [SEQ ID NO: 16]

The residue which alters the cysteine codon to a serine codon is underlined in the primer sequences.

### Example 5. Refolding of CD8αα in simplified buffer conditions.

To reduce the costs of refolding CD8αα and to eliminate some of the buffers which may be undesirable for *in vivo* use of the protein, refoldings were tested under simplified conditions.

Virtually identical refolding yields were obtained when the protein is prepared as described in Example 1 prior to refolding (i.e. CD8αα at 10 mg/ml in 2 ml Urea buffer was added to 15 ml guanidine buffer and allowed to equilibrate) but refolded in the following buffer conditions:
Refolding Buffer 2:
0-10 mM Tris-HCl (pH8.0)
0-0.1 mM PMSF
6.5 mM Cysteamine
3.7 mM Cystamine.

### Example 6. Expression of CD8αα fused to a biotinylation tag and the production of multimeric CD8αα protein complexes

The expression vector for the soluble CD8α gene was also modified to express CD8α as a fusion to the "biotinylation tag" which is a substrate for the bacterial enzyme Bir A (Schatz, 1993). The following DNA oligo (corresponding to the "antisense" strand) was used together with the sense primer described in Example 1 for a PCR reaction to generate the CD8α fusion gene: 
5' - GGG GGA AGC TTA ATG CCA TTC GAT TTT CTG AGC TTC AAA
AAT ATC GTT CAG ACC ACC ACC GGA TCC TGG CGT CGT GGT GGG
CTT CGC TGG CAG GAA GAC - 3' [SEQ ID NO: 17]
 thus adding the following protein sequence (in one letter code) to the expressed CD8α polypeptide:

GSGGGLNDIFEAQKIEWH [SEQ ID NO: 18].

The tagged CD8α protein (CD8α-BT) was expressed at similar levels to CD8α and refolded as a dimer with similar yield under the same conditions which were used for CD8α. The functional validity of the tagged protein was demonstrated by Surface Plasmon Resonance experiments in which the CD8αα-BT was immobilised by means of the biotin-streptavidin interaction and was shown to bind soluble class I MHC molecules specifically.

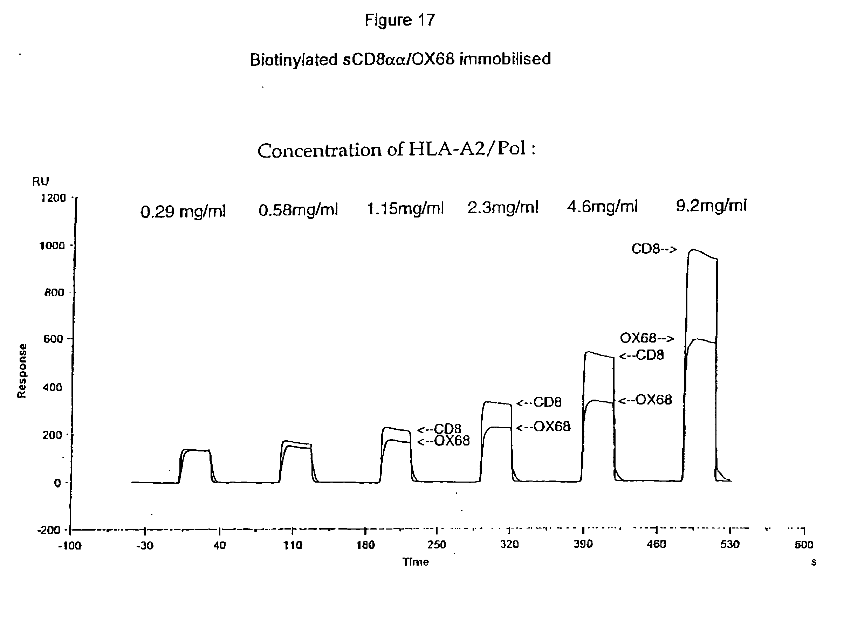

In more detail, the CD8α carrying a biotinylation tag sequence was expressed as described in *E.coli*, refolded, purified and biotinylated with the BirA enzyme (Schatz, 1993). The biotinylated SCD8αα was immobilised on the surface of a Biacore streptavidin-coated flowcell producing a change in the refractive index of 3700 response units (RU). The monoclonal antibody OX68 was immobilised on a different flowcell producing a change in the refractive index of 3400 RU. The biotinylated sCD8αα was passed in succession over the two flowcells, the OX68 antibody-coated flowcell serving as control for the CD8-coated flowcell. The soluble form of the MHC complex HLA-A2 folded with the Pol peptide of HIV-1 (HIV-1 polymerase residues 476-484 with the peptide sequence in one-letter code: ILKEPVHGV [SEQ ID NO: 12]) was flowed over the surfaces at the concentrations indicated in Figure 17. The profiles of the changes in RU from the two flowcells were overlaid (Figure 17). At every concentration, the change in RU was higher in the flowcell coated with sCD8αα than in the flowcell coated with OX68. The respective profiles from the two flowcells are indicated at the four highest concentrations of HLA-A2 complex. As shown in Figure 17, specific binding to the tagged, biotinylated and immobilised CD8 was observed. Thus, this form of CD8 can be immobilised as proposed with its functional integrity intact and is therefore suitable for the production of multimeric CD8 complexes.

Multimeric CD8 complexes can be expected to bind with higher stability to antigen presenting cells than the CD8αα dimer. The biotinylated CD8αα-BT protein could, for instance, be made as a tetramer of dimers by attachment to avidin or streptavidin, 'or attached to various types of avidin- or streptavidin-coated beads, or bound to the surface of liposomes made with derivatised lipids. In this way CD8 higher-order multimerisation particles would be obtained which would bind with significant avidity to antigen presenting cells and therefore act as efficient suppressors of the cytotoxic T cell response.

### Production of CD8 tetramers and CD8-coated beads.

In order to tetramerise the biotinylated CD8αα, extravidin (Sigma) is added at a 1:4 molar ratio. Fluorescently labelled extravidin is used for cell-labelling experiments. A step-wise addition is employed to achieve saturation of the extravidin, allowing for some incompleteness in the biotinylation reaction and some inaccuracy in the protein determinations. 15 minutes on ice is allowed between each addition of extravidin for binding, followed by at least overnight at 4°C after the final addition. Tetramerisation is confirmed by gel filtration of a small sample of the solution on a calibrated Superdex 200 column (Pharmacia). CD8 tetramer solution is then stored at 4°C in the presence of protease inhibitor cocktail and 0.05% sodium azide.

A similar approach can be used to coat various types of beads or other solid supports with soluble CD8. Avidin/streptavidin-coated beads can be obtained from commercial sources (for instance, Dynabeads from DYNAL, Oslo, Norway, or MACS from Miltenyi Biotec Ltd., Bergisch Gladbach, Germany) and are available in a wide range of sizes from approximately 4.5µm-65nm in diameter. Immobilisation of MHC-peptide complexes on Dynabeads through Biotin-Streptavidin has previously been described (Vessey *et al*., 1997). Purified biotinylated protein is incubated with streptavidin-coated beads for a period of time e.g. 30 mins at 4°C after which the beads are washed to remove unbound protein. These MHC-peptide coated beads elicited an antigen-specific response when used to stimulate a cell line expressing TCR. Similarly, tetramers of CD8, or monomeric biotinylated soluble CDB, can be immobilised on avidin/streptavidin-coated beads, or non-biotinylated CD8 can be immobilised by means of anti-CD8 antibody coating or by direct chemical crosslinking or by other appropriate means.

### Production of liposomes and drug packaging

Lipids and other components, sterile and endotoxin tested, are commercially available from a number of sources, for instance from Sigma Chemical Company or Avanti Polar Lipids Inc., USA.

Liposomes are prepared from a mixture of vesicle-forming lipids and biotinylated vesicle-forming lipids. A variety of suitable methods exist for liposome formation. Biotinylated CD8 is then linked to the exterior of the liposomes via a suitable linking agent such as avidin, streptavidin or extravidin. Detectable labels and/or reagents such as therapeutic agents, if desired are incorporated into the membrane itself or entrapped in the aqueous volume within the membrane.

### Example 7. Residues which might be mutated in order to alter the affinity of CD8αα for MHC.

The affinity of the CD8αα dimer for MHC may possibly be increased by substitution of one or more of the residues which form contacts to MHC. An increased affinity may increase the ability of the sCD8αα to inhibit cytotoxic T cell responses since the occupancy of the sCD8αα molecule on the MHC molecules on the surface of antigen presenting cells would be expected to be higher. It is envisaged that substitutions of one or more from the following list of residues, numbers referring to the amino acid positions in the mature CD8α protein sequence, could increase the affinity of CD8αα for MHC:

| | |
|---|---|
| Arginine 4 | Serine 31 |
| Lysine 21 | Serine 34 |
| Valine 24 | Tyrosine 51 |
| Leucine 25 | Glutamine 54 |
| Leucine 26 | Asparagine 55 |
| Serine 27 | Aspartate 75 |
| Asparagine 28 | Leucine 97 |
| Proline 29 | Asparagine 99 |
| Threonine 30 | Serine 100 |

See (Gao *et al*., 1997) for list of contact residues as deduced from the CD8αα/HLA-A2 structure.

Substitution of amino acid residues is routine and can be carried out using known techniques. Substituted CD8α can then be tested for binding to MHC using techniques described herein, and/or tested for a T cell inhibition effect by incorporating it into a CD8 dimer and performing a cytotoxic T cell assay, for example as decribed in Example 2.

### Example 8. Murine soluble CD8αα for animal experiments.

The homology between CD8 and the regions of MHC molecules contacted by CD8 makes it possible to test the immuno-inhibitory effect of the human CD8 in animal experiments (see also Example 10. However, it is envisaged that a murine sCD8αα will be employed for certain experiments in mouse systems. The murine sCD8αα may be of similar design to the human molecule described herein. The murine molecule will be particularly useful for the study of the long-term effects of administering sCD8αα. Although the human and murine CD8α molecules are very similar in sequence, secondary immune reactions directed against the human molecule could affect its function during long-term studies in the mouse.

A murine CD8α gene for expression in *E.coli*, and refolding into a soluble dimeric form similar to the human sCD8αα, has been generated by the polymerase chain reaction method (PCR) employing murine CTL cDNA as template and the following primer sequences designed to match the murine CD8α (Lyt-2; (Nakauchi *et al.,* 1985)):
sense primer: 
Nde I
M K P Q A P E L R I
5'- GAG GAG GAG CAT ATG AAA CCA CAA GCA CCT GAA CTA CGA ATC
F P K K M D A
TTT CCA AAG AAA ATG GAC GCC -3' [SEQ ID NO: 19]
antisense primer :

HindIII
5'- ggg gag gga agc tta ctt ggt agt agt aga gtt cac -3'
[SEQ ID NO: 20]

The bases constituting restriction sites, introduced for cloning purposes, and bases altered in the sense primer in order to increase expression in *E.coli* are underlined.

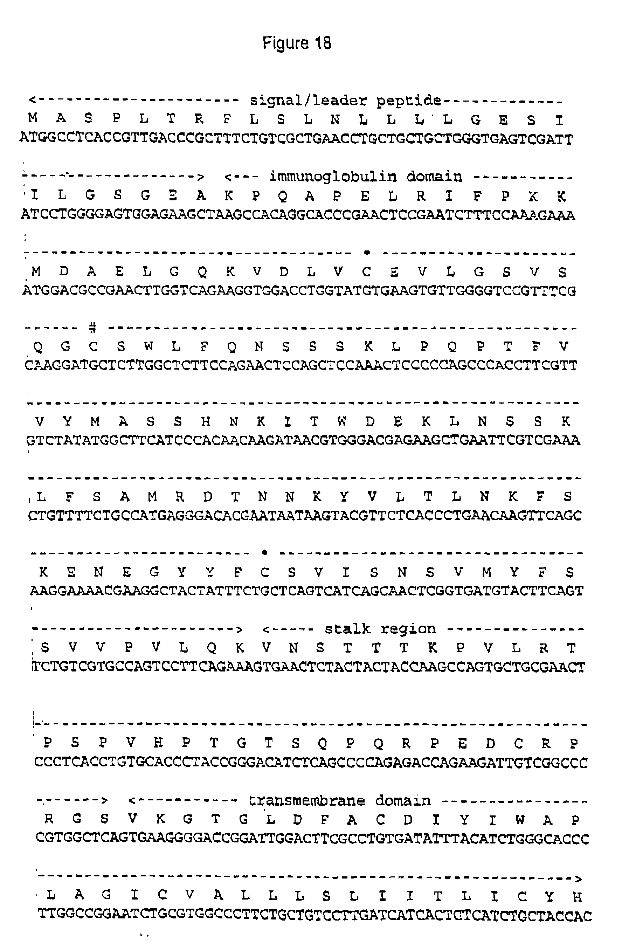

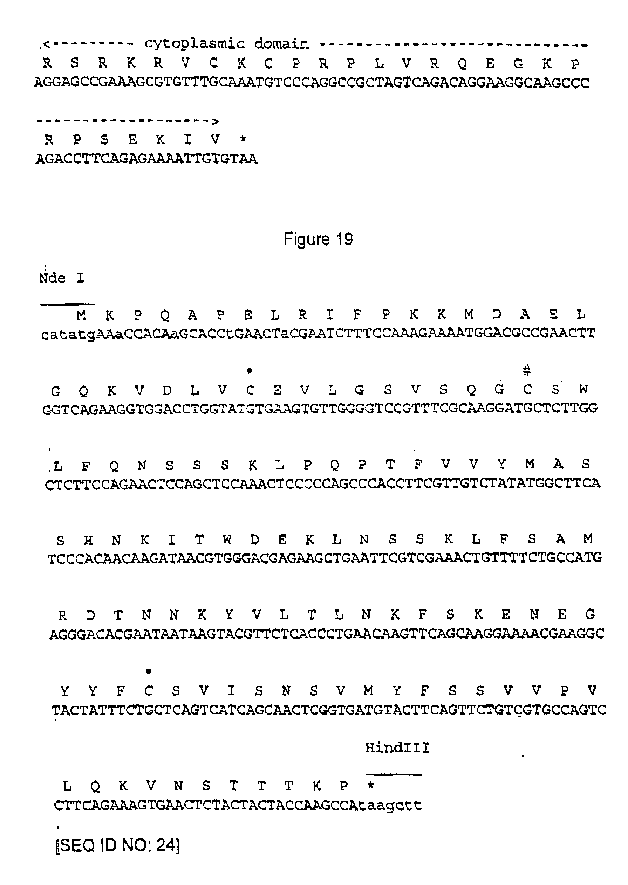

Figure 18 shows the cDNA sequence, the protein sequence, and the domain organisation of the murine CD8α (database accession no. M12052). Figure 19 shows the DNA sequerice of the DNA fragment produced in the PCR reaction and the deduced protein sequence of the recombinant, soluble murine CD8α. It is envisaged that variations of the soluble murine CD8α as described herein for the soluble human CD8α may also be generated and used in animal experiments. Such variants may be for example one or a few amino acids longer or shorter at the N-terminus, or one or a few amino acids longer or shorter at the C-terminus, than the protein depicted here. Other silent mutations (that is, DNA mutations which do not alter the protein sequence) in addition to or instead of those indicated herein may also be introduced in the 40 most 5' bases of the expression cassette shown in Figure 19 with the aim of increasing expression in *E.coli.*

Production of proteins in which the unpaired cysteine residue indicated in Fig. 18 and Fig. 19 is mutated in order to increase refolding efficiency is also envisaged.

### Legends to Figures 18 and 19

Figure 18. cDNA sequence of mouse CD8α (bottom sequence) with protein sequence (on top) indicated in one-letter code. The predicted extent of the signal sequence, immuglobulin-like domain, membrane-proximal "stalk" region, transmembrane domain and cytoplasmic domain are indicated over the protein sequence (Nakauchi et *al*., 1985; Nakauchi *et al*., 1987). The two cysteine residues which are believed to form the intrachain disulfide bond are indicated with •. The cysteine residue which is believed to be unpaired, and which may be mutated in order to increase protein yield as suggested for the human CD8α (see Example 4) is indicated with #.

Figure 19. DNA sequence (bottom sequence) of cassette for expressing mouse CD8α in *E*.*coli*. The protein sequence (on top) is indicated in one-letter. code. The two cysteine residues which are believed to form the intrachain disulfide bond are indicated with •. The cysteine residue which is believed to be unpaired, and which may be mutated in order to increase protein yield, as suggested for the human CD8α (see Example 4), is indicated with #. Recognition sites for restriction enzymes with which the cassette, generated by PCR, can be cloned into a suitable expression vector, for instance pGMT7 (Studier *et al*., 1990), are also indicated. Bases which are not part of the original DNA sequence, and which have been added to encode a start codon, allow subcloning, or have been mutated to increase bacterial expression, are shown in small letters.

### Example 9. Determination of binding affinity of soluble CD8 to MHC

A surface plasmon resonance (SPR) technique may be used to study the affinity of the interaction between soluble CD8 and MHC class I -peptide complex. This may be useful for example to determine whether a soluble CD8 molecule substituted at one or more amino acid residues has an increased or decreased binding affinity compared to non-substituted soluble CD8.

SPR studies are performed using a BlAcore™ 2000 (BlAcore AB, St Albans, UK) in HBS (BlAcore AB). HBS contains 10 mM HEPES (pH 7.4), 150 mM NaCl, 3.4 mM EDTA and 0.005% Surfactant P20. Streptavidin (Sigma) is covalently coupled to Research Grade CM5 sensor chips (BlAcore) via primary amines using the Amine Coupling kit (BlAcore). For coupling, the streptavidin is dissolved in 10 mM sodium acetate (pH 5.5) and injected at 0.5 mg/ml. Immobilisation levels range from 6000 to 11000 Response Units. Biotinylated HLA-A2 and control proteins are then immobilised by injection at 0.05 to 0.15 mg/ml for 0.5 to 10 min over streptavidin-coupled surfaces. Biotinylated mouse and rat monoclonal antibodies are used as control proteins.

Soluble CD8 which binds to the HLA-A2 will show a larger response when injected over the surface presenting immobilised HLA-A2 than when injected over the control surface. Binding affinity of a soluble CD8 for HLA can be measured.

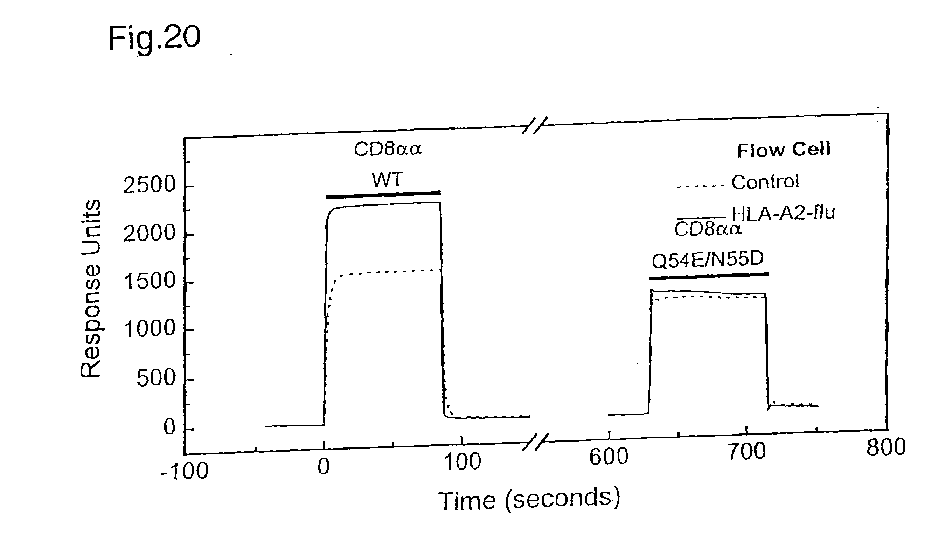

### Figure 20. CD8αα binds specifically to immobilised HLA-A2.

Similar concentrations (∼300 µM) of wild type CD8αα or the CD8αα mutant Q54E/N55D were injected (solid bars) for 90 s through flow cells with HLA-A2 *(solid trace*) or a control protein *(dotted trace)* immobilised. HLA-A2 presented an influenza matrix peptide (HLA-A2-flu, see Table 1). This experiment was performed at 25°C using a flow rate of 5 µl min⁻¹ with ∼2000 RU of HLA-A2 or a control protein immobilised. The high background responses seen in the control flow-cell reflect the high. refractive index of the injected CD8αα samples, a consequence of very high protein concentrations. CD8αα showed a larger response when injected over a surface presenting immobilised HLA-A2 than a control, indicating specific binding. The CD8αα variant did not bind, confirming the specificity of this interaction.

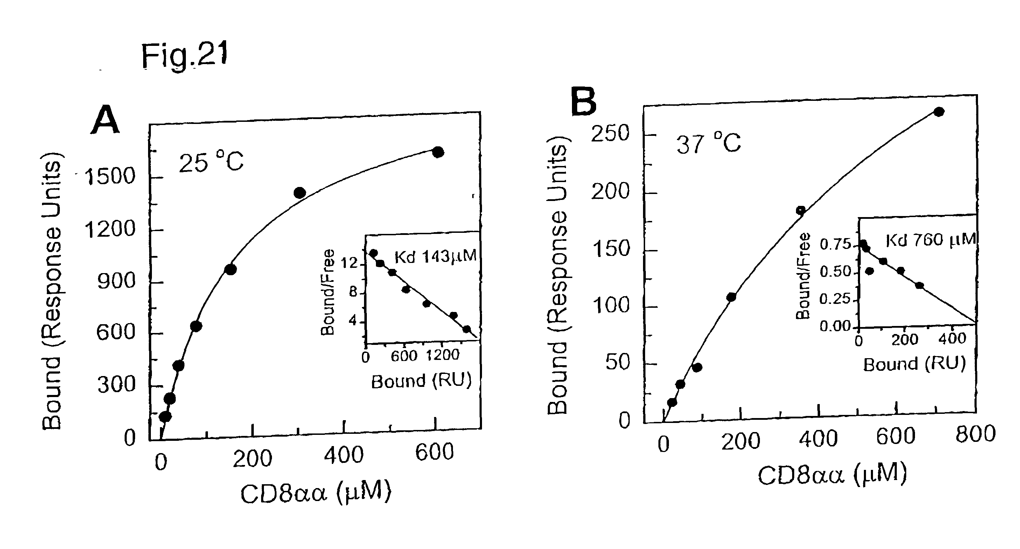

### Figure 21. The affinity of CD8αα binding to HLA-A2.

The affinity of the CD8αα/HLA-A2 interaction was determined by equilibrium binding analysis. CD8αα was injected at the indicated concentrations for 30-60 s through flow cells with either HLA-A2-flu or an irrelevant control protein immobilised. Binding was calculated as the difference between the responses in the HLA-A2-flu and control flow cells. This experiment was performed at both (A) 25°C and (B) 37°C. The solid lines represent non-linear fits of the Langmuir binding isotherm to the data. These yielded K_{d} values of 156 µM and 736 µM for (A) and (B), respectively. The insets show Scatchard plots of the same data; the Kd values shown were obtained by linear regression. The flow-rate was 5-10 µl/min with ∼9000 and ∼1900 RUs of HLA-A2-flu immobilised in (A) and (B), respectively. Similar affinities were measured for CD8αα binding to two different peptide/HLA-A2 complexes (Table 1).

### The kinetics of CD8αα binding HLA-A2

Since preliminary analysis indicated that the kinetics of the CD8αα/MHC-peptide interactions are extremely rapid, data were collected at 0.1 second intervals, the fastest rate possible on the BlAcore. When CD8αα was injected over HLA-A2 at 25°C, equilibrium was reached within 0.4 seconds, making direct determination of the association rate constant (kₒₙ) impossible. Dissociation was also extremely fast, with the response returning to baseline within 0.4 seconds of the end of the injection. Because the dissociation time was of the same order as the time taken to wash protein from the flow cell, the flow-rate was increased to 1.67 µl.s⁻¹, the maximal rate possible on the BlAcore. At this flow-rate, CD8αα dissociated with an apparent k_{off} of 18 s⁻¹. The rate at which the background response falls in the control flow cell (∼24 s⁻¹) provides an estimate of the washing time. This agrees well with the theoretical value (∼28 s⁻¹) calculated for this flow rate [flow rate ö flow cell volume (∼0.06 µL)]. The true k_{off} for the CD8αα/MHC class I interaction may well be greater than 18 s⁻¹ because rebinding following dissociation, which is commonly seen in BlAcore experiments, could not be excluded. In addition, the k_{off} at 37°C is likely to be higher than at 25°C because the affinity is ∼5 fold lower (Table 1). If the k_{off} is ≥ 18 s⁻¹ and the Kd is 126 µM ( at 25°C), the kₒₙ can be calculated to be ≥ 140000 M⁻¹.s⁻¹. The kₒₙ is typical of protein-protein interactions, and indicates that the low affinity of the CD8αα/MHC class 1 interaction is not a consequence of an unusually slow kₒₙ.

**Table 1.**

| **Equilibrium binding affinity of CD8αα binding to peptide/HLA-A2.** | | |
|---|---|---|
| Ligand* | Temp(°C) | Kd (µM)** |
| HLA-A2-flu | 25 | 159 ± 18 (n = 5) |
| HLA-A2-flu | 37 | 629 ± 151 (n=2) |
| HLA-A2-pol | 25 | 126 ± 34 (n = 7) |
| HLA-A2-pol | 37 | 712 (n = 1) |

| | | |
|---|---|---|
| *HLA-A2-flu: HLA-A2 plus Influenza matrix protein 58-66 (GILGFVFTL [SEQ ID NO: 21]); HLA-A2-pol: HLA-A2 plus HIV-1 polymerase 476-484 (ILKEPVHGV [SEQ ID NO:12]). | | |
| **Mean ± SD or, for n = 2, mean ± range of n independent determinations. | | |

### Example 10. Inhibition of CTL priming and activity by sCD8αα in vivo.

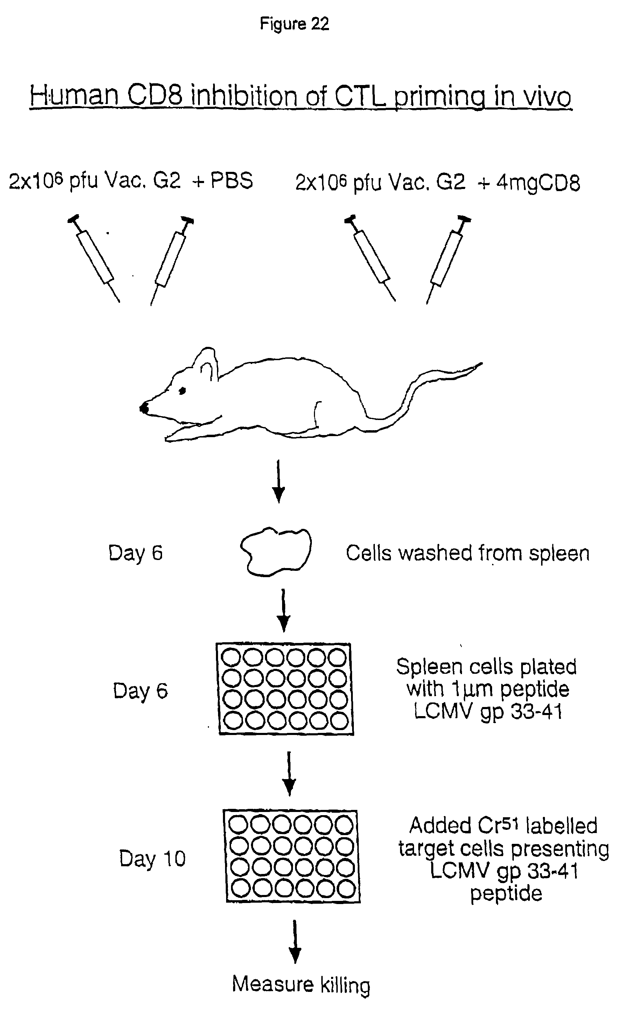

The ability of sCD8αα to inhibit a cellular immune response *in vivo* was tested by two experiments in the mouse, assaying the influence of sCD8αα on the response to an epitope encoded in a vaccinia virus (see figure 22 for schematic design). All results are presented as the average values from five individual determinations of each lysis level.

### Materials and reagents:

Mice : In both experiments mice of strain Black 6 (wildtype laboratory mouse strain) were used.

Virus : Vaccinia virus strain G2 (vvG2) is a recombinant virus strain. Infection of cells with Vaccinia virus generally results in the MHC restricted presentation of a number of vaccinia peptide epitopes which are poorly characterised. Infection with strain vvG2, however, also results in the presentation of a peptide epitope, corresponding to amino acids 33-41 (sequence in one-letter code: KAVYNFATC [SEQ ID NO: 22]), from the LCM virus G2 glycoprotein. In mice the G2 epitope is presented by the MHC molecule D^{b}.

Target cells : MC57 antigen presenting cells (Leist *et al*., 1987) express mouse MHC molecules K^{b} and D^{b}.

### Experiment 1:

Mouse 1 (M1) was injected, intraveneously, at Day 0 with 2x10⁶ pfu (plaque forming units) Vaccinia virus strain G2 plus 200 µl phosphate buffered saline (PBS, standard physiological saline buffer).

Mouse 2 (M2) was injected, intraveneously, at Day 0 with 2x10⁶ pfu Vaccinia virus strain G2 plus 200 µl sCD8αα (20mg/ml) in PBS buffer.

There were no apparent side effects in either animal.

To assay CTL activity a modification of a previously described procedure was used (Leist *et al* 1987):

On Day 6, the spleens were removed from the mice and lymphocytes washed out. Spleen cells were cultured in 24-well plates with conA supernatant. Synthetic peptide corresponding to the G2 33-41 epitope was added to the wells and lymphocytes allowed to proliferate for four days.

On Day 10, T cells were counted and CTL killing assays were performed as follows. Chromium⁵¹ -labelled MC57 cells were prepulsed with G2 33-41 peptide for one hour, then mixed with the cultured mouse lymphocytes at the indicated effector to target ratios. Chromium⁵¹ counts from wells of MC57 cells lysed by addition of detergent were set at 100% lysis.

### Experiment 2:

Mouse 3 (M3) was treated identically to M1 except that injections were performed intraperitonally.

Mouse 4 (M4) was treated identically to M2 except that injections were performed intraperitonally.

Again, there were no apparent side effects.

**Table 2.**

| E:T | M1 (PBS) | M2 (CD8) | M3 (PBS) | M4 (CD8) |
|---|---|---|---|---|
| 21.1 | 76% | 42% | 89% | 43% |
| 7:1 | 73% | 35% | 66% | 30% |

### In vitro control experiment:

To verify that the CTL response against G2 33-41 is CD8 dependent, and inhibitable by sCD8αα, the effect of the soluble CD8 was tested in a lysis assay employing the CTL cultured from Mouse 1. As shown in Table 3, the mouse CTL response against G2 33-41 is clearly inhibitable by both anti-CD8 antibody and sCD8αα.

**Table 3.**

| | | | |
|---|---|---|---|
| Inhibition of M1 CTL response by anti-CD8 mAb YTS 169.4 (Cobbold *et al*., 1984) and by sCD8αα. Effector to target ratio (E:T) was 20:1 in all experiments. 100 µg anti-CD8 mAb YT62.1 or sCD8αα in 25 µl were added into 200 µl assays. | | | |
| Addition | PBS | YT62.1 | sCD8αα |
| Lysis level | 82% | 46% | 44% |

### Experiment 3:

Mice 5 and 6 (M5 and M6; control animals) were treated identically to M3 except that two injections of PBS buffer were administered, one five hours prior to infection with vaccinia virus G2, and a further injection 24 hours after the first one.

Mouse 7 and 8 (M7 and M8) were treated identically to M4 except that two injections of sCD8αα were administered, one five hours prior to infection with vaccinia virus G2, and a further injection 24 hours after the first one. As with experiments. 1 and 2, no apparent side effects were observed.

On day 6, after the cells were washed out of the spleens, half the wells with lymphocyte cultures were grown in the presence of ConA supernatant while the other half were grown without. This was done to test whether the effects of sCD8αα were more clearly detectable without the addition of ConA supernatant. The ConA supernatant contains several cytokines and therefore stimulates both specific and unspecific lysis activity, thus amplifying the lysis which is observed after culturing. This could contribute to obscuring the inhibitory effect of sCD8αα.

As seen in Table 4, the sCD8αα has in both animals had a clear inhibitory effect on the proliferation of cytotoxic T cells specific for the G2 peptide. The inhibitory effect is, as expected, slightly clearer in the cultures grown without ConA supernatant.

**Table 4.**

| Lysis levels of four sets of spleen cultures grown from each of M5-M8. Each lysis level was determined in duplicate. | | | | | |
|---|---|---|---|---|---|
| | | Exp.1 | Exp.2. | Exp.3 | Exp.4 |
| M5 | -ConA | 14% | 21% | 31% | 35% |
| M6 | -ConA | 21% | 20% | 33% | 45% |
| | | | | | |
| M7 | -ConA | -1% | -1% | 4% | 11% |
| M8 | -ConA | -4% | 0% | 3% | 10% |
| | | | | | |
| M1 | +ConA | 19% | 28% | 25% | 33% |
| M2 | +ConA | 19% | 33% | 56% | 38% |
| | | | | | |
| M7 | +ConA | 2% | 7% | 7% | 20% |
| M8 | +ConA | 7% | 12% | 21% | 24% |

The "negative" lysis observed in two of the spleen cultures from M7 and M8 is due to complete inhibition of CTL and the subtraction of "spontaneous lysis" from all of the results. This phenomenon is common in CTL assays.

### References

**Baert,** F., and Rutgeerts, P. (1997). Immunomodulator therapy of inflammatory bowel disease. Acta Clin Belg 52, 251-7.

**Boursier,** J.P., Alcover, A., Herve, F., Laisney, I. and Acuto, O: (1993) J. Biol. Chem. *268*, 2013-2020.

**Chatenoud,** L. (1998). Tolerogenic antibodies and fusion proteins to prevent graft rejection and treat autoimmunity. Mol Med Today *4*, 25-30.

**Choski,** S., Jameson, B.A. and Korngold, R. (1998). Nature Medicine vol.4 no.3, 309-314.

**Cobbold,** S. P., Jayasuriya, A., Nash, A., Prospero, T. D., and Waldmann, H. (1984). Therapy with monoclonal antibodies by elimination of T-cell subsets in vivo. Nature *312*, 548-51.

**Gao**, G. F., Tormo, J., Gerth; U. C., Wyer, J. R., McMichael, A. J., Stuart, D. I., Bell, J. I., Jones, E. Y., and Jakobsen, B. K. (1997). Crystal structure of the complex between human CD8alpha(alpha) and HLA-A2. Nature *387*, 630-4.

**Garcia**, K.C., Scott, C.A., Brunmark, A., Carbone, F.R., Peterson, P.A., Wilson, I.A. and Teyton, L. (1996) Nature *384* 577-581.

**Leahy**, D. J., Axel, R., and Hendrickson, W. A. (1992). Crystal structure of a soluble form of the human T cell coreceptor CD8 at 2.6 A resolution. Cell *68*, 1145-62 Issn: 0092-8674.

**Leist**, T. P., Cobbold, S. P., Waldmann, H., Aguet, M., and Zinkernagel, R. M. (1987). Functional analysis of T lymphocyte subsets in antiviral host defense. J Immunol *138*, 2278-81.

**McKendry**, R. J. (1997). The remarkable spectrum of methotrexate toxicities. Rheum Dis Clin North Am *23*, 939-54.

**Nakauchi**, H., Nolan, G. P., Hsu, C., Huang, H. S., Kavathas, P., and Herzenberg, L. A. (1985). Molecular cloning of Lyt-2, a membrane glycoprotein marking a subset of mouse T lymphocytes: molecular homology to its human counterpart, Leu-2/T8, and to immunoglobulin variable regions. Proc Natl Acad Sci U S A *82*, 5126-30.

**Nakauchi,** H., Tagawa, M., Nolan, G. P., and Herzenberg, L. A. (1987). Isolation and characterization of the gene for the murine T cell differentiation antigen and immunoglobulin-related molecule, Lyt-2. Nucleic Acids Res *15*, 4337-47.

**Ortiz,** Z., Shea, B., Suarez Almazor, M. E., Moher, D., Wells, G. A., and Tugwell, P. (1998). The efficacy of folic acid and folinic acid in reducing methotrexate gastrointestinal toxicity in rheumatoid arthritis. A metaanalysis of randomized controlled trials. J Rheumatol *25*, 36-43.

**Purbhoo,** M.A. *et al*. Copresentation of natural HIV-1 agonist and antagonist ligands fails to induce the T cell receptor signaling cascade. Proc. Natl. Acad. Sci. USA *95*, 4527-4532 (1998).

**Schatz,** P. J. (1993). Use of peptide libraries to map the substrate specificity of a peptide-modifying enzyme: a 13 residue consensus peptide specifies biotinylation in Escherichia coli. Biotechnology N Y *11*, 1138-43.

**Sibilia,** J., Liote, F., and Mariette, X. (1998). Lymphoproliferative disorders in rheumatoid arthritis patients on low-dose methotrexate. Rev Rhum Engl Ed *65*, 267-73

**Singer,** N. G., and McCune, W. J. (1998). Update on immunosuppressive therapy. Curr Opin Rheumatol *10*, 169-73.

**Studier,** F. W., Rosenberg, A. H., Dunn, J. J., and Dubendorff, J. W. (1990). Use of T7 RNA polymerase to direct expression of cloned genes. Methods Enzymol *185*, 60-89 Issn: 0076-6879.

**Verhoeven,** A. C., Boers, M., and Tugwell, P. (1998). Combination therapy in rheumatoid arthritis: updated systematic review. Br J Rheumatol *37*, 612-9.

**Vessey,** S. J. R., Barouch, D. H., McAdam, S. N., Tussey, L. G.,

Davenport, M. A., O'Callaghan, C. A., I., B. J., McMichael, A. J., and Jakobsen, B. K. (1997). Engagement of a T cell receptor by major histocompatibility complex irrespective of peptide. Eur J Immunol *27*, 879-885.

## Claims

1. The use of a soluble CD8αα or αβ molecule which comprises a correctly-folded immunoglobulin domain in the manufacture of a medicament for immunosuppressive therapy.

2. The use of a multimer of two or more soluble CD8αα or αβ molecules in the manufacture of a medicament for immunosuppressive therapy.

3. The use according to claim 1 or claim 2, wherein the soluble CD8 is human CD8.

4. The use according to claim 1, 2 or 3, wherein the soluble CD8 comprises all or a substantial part of the native CD8 immunoglobulin domain, optionally substituted at one or more amino acid residues.

5. The use according to claim 4, wherein the soluble CD8 further comprises a fragment of the membrane-proximal stalk region of CD8.

6. The use according to claim 5, wherein the soluble CD8 comprises residues 1-120 of mature CD8.

7. The use according to any one of claims 1 and 3 to 6, wherein the soluble CD8 is provided as a multimer of two or more CD8αα or αβ molecules.

8. A composition comprising a soluble form of a CD8αα or αβ molecule which comprises a correctly-folded immunoglobulin domain, together with a pharmaceutically acceptable diluent, excipient or carrier, for immunosuppressive therapy.

9. A composition according to claim 8, wherein the soluble CD8 is human CD8.

10. A composition according to claim 8 or claim 9, wherein the soluble CD8 comprises all or a substantial part of the native CD8 immunoglobulin domain, optionally substituted at one or more amino acid residues.

11. A composition according to claim 10, wherein the soluble CD8 further comprises a fragment of the membrane-proximal stalk region of CD8.

12. A composition according to any one of claims 8 to 10, wherein the soluble CD8 is provided as a multimer of two or more CD8 molecules.

13. A multimer of soluble CD8, comprising two or more CD8αα or αβ molecules attached to one another via a linker molecule.

14. A multimer according to claim 12, wherein the soluble CD8 is human CD8.

15. A multimer according to claim 12 or claim 13, wherein the soluble CD8 comprises all or a substantial part of the native CD8 immunoglobulin domain, optionally substituted at one or more amino acid residues.

16. A multimer according to claim 14, wherein the soluble CD8 further comprises a fragment of the membrane-proximal stalk region of CD8.

17. A protein having the sequence as shown in figure 1b, said protein folded as a dimer and having the property of inhibiting the action of cytotoxic T lymphocytes to kill target cells.

18. A protein which differs from that shown in figure 1b in one or more of the following respects:
(i) methionine absent at the N-terminus;
(ii) 1-15, amino acid residues absent from the N-terminus;
(iii) part or all of the sequence leu - leu - leu - his - ala - ala - arg - pro-added at the N-terminus;
(iv) 1-15 amino acid residues absent from the C-terminus; but with at least a part of the region defined by amino acid residues 116 - 120 retained;
(v) part or all of the sequence - ala - pro - arg - pro - pro - thr - pro - ala added at the C-terminus;
(vi) conservative variants of one or many amino acid residues which do not materially affect the CD8 functionality of the protein;
(vii) mutations which do alter the CD8 functionality in such a way that they increase the property of inhibiting the action of cytotoxic T cell lymphocytes to kill target cells;
(viii) the addition of a protein or peptide, at the N or C terminus for the purposes of purification;
(ix) the provision of a label for detection;
said protein folded as a dimer and having the property of inhibiting the action of cytotoxic T lymphocytes to kill target cells.

19. A protein according to claim 18, which is a soluble CD8 molecule containing a substantial part of the extracellular region of CD8, including the immunoglobulin domain and a fragment of the membrane-proximal stalk region, which CD8 molecule is not disulphide-linked between the two chains of the molecule.

20. A complex of a protein according to any one of claims 17 to 19 with HLA-A2.

21. A complex as claimed in claim 20 in crystalline form with a stoichiometry of one protein dimer to one HLA - A2 peptide unit.

22. A method of producing the recombinant protein according to any one of claims 17 to 19, which method comprises the steps of:
i) providing a CD8 derived gene which codes for a protein according to any one of claims 17 to 19 in a bacterium;
ii) effecting expression of said CD8 derived gene in said bacterium and recovering of the expressed protein from a bacterial culture;
iii) treating the expressed protein to facilitate its purification and carrying out said purification.

23. The method of claim 22 wherein the CD8 derived gene provided at step i) is modified via silent mutations designed to increase expression via the prevention of the formation of a 5' hairpin secondary structure in the expressed mRNA.

24. The method of claims 22 or 23 wherein at step iii) the treatment of the expressed protein involves solubilising the protein and treating the protein so as to cause it to fold into a form resembling its native state, which is then purified.

## Patentansprüche

1. Verwendung eines löslichen CD8-αα oder αβ-Moleküls, welches eine korrekt gefaltete Immunglobulin-Domäne aufweist, in der Herstellung eines Medikaments für eine Therapie zur Unterdrückung der Immunantwort.

2. Verwendung eines Multimer von zwei oder mehr löslichen CD8-αα oder αβ-Molekülen in der Herstellung eines Medikaments für eine Therapie zur Unterdrückung der Immunantwort.

3. Verwendung nach Anspruch 1 oder 2, wobei das lösliche CD8 menschliches CD8 ist.

4. Verwendung nach einem der Ansprüche 1, 2 oder 3, wobei das lösliche CD8 die ganze oder einen wesentlichen Teil der nativen CD8-Immunglobulin-Domäne aufweist und zwar gegebenenfalls an einem oder mehreren Aminosäureresten substituiert.

5. Verwendung nach Anspruch 4, wobei das lösliche CD8 weiters ein Fragment der membran-proximalen Stielregion des CD8 aufweist.

6. Verwendung nach Anspruch 5, wobei das lösliche CD8 die Reste 1-120 des reifen CD8 aufweist.

7. Verwendung nach einem der Ansprüche 1 und 3 bis 6, wobei das lösliche CD8 als Multimer von zwei oder mehreren CD8-αα oder αβ-Molekülen bereitgestellt wird.

8. Zusammensetzung, welche eine lösliche Form eines CD8-αα oder αβ-Moleküls enthält, die eine korrekt gefaltete Immunglobulin-Domäne zusammen mit einem pharmazeutisch annehmbaren Verdünnungsmittel, Exzipient oder Träger für eine Therapie zur Unterdrückung der Immunantwort enthält.

9. Zusammensetzung nach Anspruch 8, wobei das lösliche CD8 menschliches CD8 ist.

10. Zusammensetzung nach Anspruch 8 oder 9, wobei das lösliche CD8 die ganze oder einen wesentlichen Teil der nativen CD8-Immunglobulin-Domäne aufweist und zwar gegebenenfalls an einem oder mehreren Aminosäureresten substituiert.

11. Zusammensetzung nach Anspruch 10, wobei das lösliche CD8 weiters ein Fragment der membran-proximalen Stielregion des CD8 aufweist.

12. Zusammensetzung nach einem der Ansprüche 8 bis 10, wobei das lösliche CD8 als Multimer von zwei oder mehreren CD8-Molekülen bereitgestellt wird.

13. Multimer des löslichen CD8, welches zwei oder mehrere CD8-αα oder αβ-Moleküle enthält, die durch ein Linker-Molekül aneinander angelagert sind.

14. Multimer nach Anspruch 12, wobei das lösliche CD8 menschliches CD8 ist.

15. Multimer nach Anspruch 12 oder 13, wobei das lösliche CD8 die ganze oder einen wesentlichen Teil der nativen CD8-Immunglobulin-Domäne aufweist und zwar gegebenenfalls an einem oder mehreren Aminosäureresten substituiert.

16. Multimer nach Anspruch 14, wobei das lösliche CD8 weiters ein Fragment der membran-proximalen Stielregion des CD8 aufweist.

17. Protein mit der in Figur 1b gezeigten Sequenz, wobei das Protein als Dimer gefaltet ist und die Eigenschaft hat, die Wirkung zytotoxischer T-Lymphozyten, Zielzellen zu töten, zu hemmen.

18. Protein, das sich von dem in Figur 1b hinsichtlich eines oder mehrerer der folgenden Punkte unterscheidet:
(i) Fehlen des Methionins am N-Terminus;
(ii) Fehlen von 1-15 Aminosäureresten vom N-Terminus;
(iii) Hinzufügen von einem Teil der oder der gesamten Sequenz leu-leu-leuhis-ala-ala-arg-pro an den N-Terminus;
(iv) Fehlen von 1-15 Aminosäureresten vom C-Terminus; jedoch Zurückbehalten von zumindest einem Teil der Region, die durch die Aminosäurereste 116-120 definiert ist;
(v) Hinzufügen von einem Teil der oder der gesamten Sequenz -ala-proarg-pro-pro-thr-pro-ala an den C-Terminus;
(vi) konservative Varianten von einem oder vielen Aminosäureresten, die sich nicht wesentlich auf die CD8 Funktionalität des Proteins auswirken;
(vii) Mutationen, die die CD8 Funktionalität in einer Weise ändern, die die Eigenschaft steigert, die Wirkung zytotoxischer T-Zell-Lymphozyten, Zielzellen zu töten, zu hemmen.
(viii) Hinzufügung eines Proteins oder Peptids, an den N- oder C-Terminus zum Zwecke der Reinigung;
(ix) Versorgung mit einem Marker für die Detektion;
wobei das Protein zu einem Dimer gefaltet ist und die Eigenschaft hat, die Wirkung zytotoxischer T-Zell-Lymphozyten, Zielzellen zu töten, zu hemmen.

19. Protein nach Anspruch 18, das ein lösliches CD8-Molekül ist, welches einen wesentlichen Teil der extrazellulären Region des CD8 enthält, einschließlich der Immunglobulin-Domäne und einem Fragment der membran-proximalen Stielregion, wobei das CD8-Molekül nicht über ein Disulfid zwischen den beiden Ketten des Moleküls verbunden ist.

20. Komplex eines Proteins nach einem der Ansprüche 17 bis 19 mit HLA-A2.

21. Komplex nach Anspruch 20 in kristalliner Form, mit einem stöchiometrischen Verhältnis von einem Proteindimer zu einer HLA-A2-Peptideinheit.

22. Verfahren zur Herstellung des rekombinanten Proteins nach einem der Ansprüche 17 bis 19, welches Verfahren folgende Schritte aufweist:
(i) Bereitstellen eines von CD8 abgeleiteten Gens, welches für ein Protein nach einem der Ansprüche 17 bis 19 in einem Bakterium codiert;
(ii) Bewirken der Expression des von CD8 abgeleiteten Gens in dem Bakterium und Gewinnung des exprimierten Proteins von einer Bakterienkultur;
(iii) Behandeln des exprimierten Proteins, um seine Reinigung zu erleichtern und Ausführen der Reinigung.

23. Verfahren nach Anspruch 22, wobei das von CD8 abgeleitete, in Schritt (i) bereitgestellte Gen durch stille Mutationen verändert ist, wobei die Mutationen ausgerichtet sind, um die Expression durch Verhinderung der Bildung einer 5'-Haarnadel-Sekundärstruktur in der exprimierten mRNA zu erhöhen.

24. Verfahren nach Anspruch 22 oder 23, wobei in Schritt (iii) die Behandlung des exprimierten Proteins beinhaltet, das Protein löslich zu machen und das Protein derart zu behandeln, um es zu veranlassen, daß es sich in eine Form faltet, die seinem nativen Zustand ähnelt, wobei das Protein dann gereinigt ist.

## Revendications

1. Utilisation d'une molécule soluble de CD8 αα ou αβ, qui comprend un domaine d'immunoglobuline correctement replié dans la fabrication d'un médicament pour une thérapie d'immunosuppression.

2. Utilisation d'un multimère de deux molécules solubles de CD8 αα ou αβ ou davantage dans la fabrication d'un médicament pour une thérapie d'immunosuppression.

3. Utilisation suivant la revendication 1 ou la revendication 2, dans laquelle le CD8 soluble est un CD8 humain.

4. Utilisation suivant la revendication 1, 2 ou 3, dans laquelle le CD8 soluble comprend tout ou une partie substantielle du domaine natif d'immunoglobuline de CD8, présentant une substitution facultative au niveau d'un ou de plusieurs résidus d'acides aminés.

5. Utilisation suivant la revendication 4, dans laquelle le CD8 soluble comprend en outre un fragment de la région d'ancrage du CD8 proche de la membrane.

6. Utilisation suivant la revendication 5, dans laquelle le CD8 soluble comprend les résidus 1-120 du CD8 mature.

7. Utilisation suivant l'une quelconque des revendications 1 et 3 à 6, dans laquelle le CD8 soluble est procuré sous forme d'un multimère de deux molécules de CD8 αα ou αβ ou davantage.

8. Composition comprenant une forme soluble d'une molécule de CD8 αα ou αβ, qui comprend un domaine d'immunoglobuline correctement replié, conjointement avec un diluant, excipient ou vecteur pharmaceutiquement acceptable, pour une thérapie d'immunosuppression.

9. Composition suivant la revendication 8, dans laquelle le CD8 soluble est un CD8 humain.

10. Composition suivant la revendication 8 ou la revendication 9, dans laquelle le CD8 soluble comprend tout ou une partie substantielle du domaine natif d'immunoglobuline de CD8, présentant une substitution facultative au niveau d'un ou de plusieurs résidus d'acides aminés.

11. Composition suivant la revendication 10, dans laquelle le CD8 soluble comprend en outre un fragment de la région d'ancrage du CD8 proche de la membrane.

12. Composition suivant l'une quelconque des revendications 8 à 10, dans laquelle le CD8 soluble est procuré sous forme d'un multimère de deux molécules de CD8 ou davantage.

13. Multimère de CD8 soluble comprenant deux molécules de CD8 αα ou αβ ou davantage liées l'une à l'autre par l'intermédiaire d'une molécule de bras de liaison.

14. Multimère suivant la revendication 12, dans lequel le CD8 soluble est un CD8 humain.

15. Multimère suivant la revendication 12 ou la revendication 13, dans lequel le CD8 soluble comprend tout ou une partie substantielle du domaine natif d'immunoglobuline de CD8, présentant une substitution facultative au niveau d'un ou de plusieurs résidus d'acides aminés.

16. Multimère suivant la revendication 14, dans lequel le CD8 soluble comprend en outre un fragment de la région d'ancrage du CD8 proche de la membrane.

17. Protéine présentant la séquence telle que présentée à la Fig. 1b, ladite protéine étant repliée sous forme d'un dimère et présentant une propriété d'inhibition de l'action des lymphocytes T cytotoxiques visant à tuer des cellules cibles.

18. Protéine qui diffère de celle présentée à la Fig. 1b, au niveau d'un ou de plusieurs des aspects suivants :
(i) la méthionine est absente à l'extrémité N;
(ii) 1-15 résidus d'acides aminés sont absents à l'extrémité N;
(iii) une partie ou toute la séquence leu-leu-leu-his-ala-ala-arg-pro est ajoutée à l'extrémité N;
(iv) 1-15 résidus d'acides aminés sont absents à l'extrémité C; mais au moins une partie de la région définie par les résidus d'acides aminés 116-120 est conservée;
(v) une partie ou toute la séquence ala-pro-arg-pro-pro-thr-pro-ala est ajoutée à l'extrémité C;
(vi) des variations conservatrices d'un ou de plusieurs résidus d'acides aminés qui n'altèrent pas matériellement la fonctionnalité de CD8 de la protéine;
(vii) des mutations qui altèrent la fonctionnalité du CD8, réalisées d'une manière telle qu'elles augmentent la propriété d'inhibition de l'action des lymphocytes T cytotoxiques visant à tuer des cellules cibles;
(viii) l'addition d'une protéine ou d'un peptide, à l'extrémité N ou C à des fins de purification;
(ix) l'apport d'un marqueur pour une détection;
ladite protéine étant repliée sous forme d'un dimère et présentant une propriété d'inhibition de l'action des lymphocytes T cytotoxiques visant à tuer des cellules cibles.

19. Protéine suivant la revendication 18, qui est une molécule soluble de CD8 contenant une partie substantielle de la région extracellulaire du CD8, y compris le domaine d'immunoglobuline et un fragment de la région d'ancrage proche de la membrane, laquelle molécule de CD8 n'est pas liée par un pont disulfure entre les deux chaînes de la molécule.

20. Complexe d'une protéine suivant l'une quelconque des revendications 17 à 19, avec HLA-A2.

21. Complexe suivant la revendication 20, sous forme cristalline présentant une stoechiométrie d'un dimère protéique pour une unité peptidique de HLA-A2.

22. Procédé de production de la protéine recombinante suivant l'une quelconque des revendications 17 à 19, lequel procédé comprend les étapes de :
i) l'apport d'un gène provenant du CD8 qui code pour une protéine suivant l'une quelconque des revendications 17 à 19 dans une bactérie;
ii) la réalisation de l'expression dudit gène provenant du CD8 dans ladite bactérie et la récupération de la protéine exprimée à partir d'une culture bactérienne;
iii) le traitement de la protéine exprimée pour faciliter sa purification et la réalisation de ladite purification.

23. Procédé suivant la revendication 22, dans lequel le gène provenant du CD8 procuré à l'étape i) est modifié au moyen de mutations silencieuses conçues pour augmenter l'expression en empêchant la formation d'une structure secondaire en épingle à cheveu en 5' dans l'ARNm exprimé.

24. Procédé suivant les revendications 22 ou 23, dans lequel à l'étape iii) le traitement de la protéine exprimée implique la solubilisation de la protéine et le traitement de la protéine de manière à provoquer son repliement en une forme ressemblant à son état natif, qui est ensuite purifiée.
